# EUROPEAN PATENT SPECIFICATION

(11) **EP 3 455 249 B1**
(45) Date of publication and mention of the grant of the patent: **03.05.2023**
(21) Application number: 17725537.9
(22) Date of filing: 09.05.2017
(51) Int. Cl.: C07K 14/725, C07K 14/495

(54) **T-CELL RECEPTORS WHICH RECOGNISE FRAMESHIFT MUTANTS OF TGFBETARII**
T-ZELL-REZEPTOREN, DIE RASTERSCHUBMUTANTEN VON TGFBETARII ERKENNEN
RÉCEPTEURS DE LYMPHOCYTES T RECONNAISSANT DES MUTANTS DE DÉPHASAGE DE TGFBETARII

(30) Priority: 09.05.2016 GB 201608052
(43) Date of publication of application: 20.03.2019
(73) Proprietor: Oslo Universitetssykehus HF, 0424 Oslo (NO)
(72) Inventor: INDERBERG, Else Marit, 0276 Oslo (NO); GAUDERNACK, Gustav, 1337 Sandvika (NO); WÄLCHLI, Sèbastien, 0276 Oslo (NO); KVALHEIM, Gunnar, 0381 Oslo (NO)
(74) Representative: Dehns
(86) International application number: PCT/EP2017/061087
(87) International publication number: WO 2017/194555

(56) References cited:
- WO-A2-2015/189267
- Inderberg-Suso: "Abstract 3146: T cell therapy targeting a neoantigen reduces in vivo tumour growth | Cancer Research", , 1 August 2015 (2015-08-01), XP055393369, Retrieved from the Internet: URL:http://cancerres.aacrjournals.org/cont ent/75/15_Supplement/3146 [retrieved on 2017-07-24]

## Description

This invention relates to T-cell receptors (TCRs) which recognise frameshift mutants of Transforming Growth Factor-β Receptor II (TGFβRII). These TCRs have use in the treatment of cancer, specifically cancers which contain particular frameshift mutations of TGFβRII. The invention provides TCR molecules, nucleic acid molecules which encode such TCRs and vectors containing these nucleic acid molecules. The nucleic acid molecules and vectors provided may be used to modify immune effector cells, including notably T-cells, to express the TCR. The nucleic acid molecules and vectors may also be used to modify production host cells to produce the TCR. Such modified immune effector cells may be used in adoptive cell transfer therapy. In particular, the TCRs of the invention are based on or derived from a particular TCR, identified herein as Radium-1, which was identified in a cytotoxic T-lymphocyte (CTL) clone isolated from a clinically-responding patient immunised with a TGFβRII frameshift peptide, and which recognises the frameshift peptide neoepitope sequence RLSSCVPVA (SEQ ID NO: 1).

Worldwide, colorectal carcinoma (CRC) is the third most common cancer in men and the second most common in women, with the highest rates of CRC being seen in the West. Lynch Syndrome, or hereditary non-polyposis colorectal cancer (HNPCC), is an inherited condition in which DNA mismatch repair is impaired, resulting in microsatellite instability (MSI). Sufferers of Lynch Syndrome are at high risk of developing various cancers, including CRC. A subset of sporadic cancers (i.e. non-hereditary cancers), including colorectal and gastric cancers, also display MSI.

MSI leads to the insertion or deletion of single or di-nucleotides in short repetitive DNA sequences. When such mutations occur in genes encoding proteins they cause a shift in the reading frame of the gene (i.e. they are frameshift mutations). Such mutations generally result in the generation of truncated, non-functional proteins.

Transforming Growth Factor-β (TGF-β) proteins bind TGFβRII receptor proteins at the cell surface, activating a signalling pathway which leads to cell cycle arrest. Mutation of TGFβRII can lead to the inactivation of this pathway, contributing to carcinogenesis. Frameshift mutations which inactivate TGFβRII occur in approximately 90% of microsatellite instable (MSI+) and approximately 15 % of microsatellite stable (MSS, non-MSI+ or MSI-) colon cancers. These mutations largely occur in a mutation-vulnerable polyadenine tract in exon 3 of *TGFβRII.*

MSI+ colon cancers are considered to be more immunogenic than MSS cancers due to the generation of neopeptides (i.e. peptides with sequences not naturally found in the individual, which are, therefore, recognised by the immune system as "non-self") created by frameshift mutations in genes containing microsatellite repeats within the coding regions of their transcribed sequences. Lynch Syndrome patients, and patients with the MSI+ subtype of sporadic colon cancers, have an improved prognosis compared to other sporadic colon cancer patients. The number and presence of certain frameshift mutations in MSI+ colon cancers correlate with the increased density of tumour-infiltrating lymphocytes (TILs) characterizing these cancers. The correlation between an increased density of TILs in MSI+ colorectal cancers and improved survival compared to non-MSI+ colorectal cancers is also well established. The enhanced host immune response could, at least partially, explain the improved prognosis of these cancers. These observations suggest that some patients with MSI+ CRC may benefit from immunotherapy targeting products of frameshift mutations in genes such as TGFβRII.

T-cell epitopes have been identified within these frameshift mutation-derived neopeptides. The TGFβRII "-1A" mutation, wherein one adenine residue is lost from the above-mentioned polyadenine tract in exon 3 of *TGFβRII,* is an example of a mutation which results in the production of neopeptides which contain T-cell epitopes, including both CD4⁺ and CD8⁺ T-cell epitopes.

T-cell epitopes are recognised by TCRs, which are protein complexes which protrude from the cell membrane of a T-cell. Most TCRs comprise an α- and a β-chain, both of which consist of a variable region and a constant region. The variable region is located at the N-terminus of the chain, and is wholly extracellular; the constant region is located at the C-terminus of the chain, and consists of an extracellular domain, a transmembrane domain and a short cytoplasmic domain. TCR chains are encoded and synthesised in an immature form, with an N-terminal signal (or leader) sequence. This sequence forms the N-terminus of the variable region of an α- or β- TCR chain when it is synthesised. Following synthesis of the TCR chain, the signal sequence is cleaved, and so is not present in a mature TCR located at the cell surface. Recently, soluble TCRs (sTCRs) have been developed, which comprise the variable regions, and the extracellular domains of the constant regions, of the α- and β-chains as present in native TCRs, but lack the transmembrane and cytoplasmic domains of the constant regions. Soluble TCRs may be expressed by any cell, and are secreted.

The variable region of an α- or β-chain comprises three hypervariable, complementarity determining regions (CDRs). These CDRs determine the specificity of the TCR, with CDR3 (that is, the third CDR from the N-terminus) being the most important CDR in determining TCR specificity. The sections of the variable regions of TCR chains which do not form the CDRs are known as framework regions. A TCR variable region contains four such framework regions. Framework region 1 is N-terminal to CDR1; framework region 2 links CDR1 and CDR2; framework region 3 links CDR2 and CDR3; framework region 3 links CDR3 to the constant region of the TCR chain. These framework regions are much less variable than the CDRs, and form a scaffold for the CDRs. The sequence of the framework regions is important for TCR function, as they determine the overall structure of the variable region of a TCR chain. This structure must hold the CDRs in the correct orientations and relative positions for them to bind the target antigen.

The variable region of a TCR thus binds a target antigen, TCR antigens being proteins. The specific part of the antigen bound by the TCR is the T-cell epitope. T-cell epitopes are short antigen fragments, generally peptides between 8 and 17 amino acids in length. The relevant antigen fragment is presented to the TCR by a Major Histocompatibility Complex (MHC). Upon binding the antigen, the TCR activates a signal transduction pathway which activates the T-cell to initiate an immune response.

There are two classes of MHCs: Class I and Class II. Class I MHCs are expressed by all nucleated cells; Class II MHCs are expressed only by professional antigen-presenting cells (APCs), such as dendritic cells. The function of all MHCs is to present short peptide segments for recognition by T-cells. A Class I MHC presents peptide fragments from within the cell on which it is expressed, and is recognised by CD8⁺ T-cells (cytotoxic T-cells). If a CD8⁺ T-cell recognises a peptide presented by a Class I MHC as an antigen, the T-cell triggers apoptosis of the cell on which that Class I MHC is expressed. A Class II MHC presents peptide fragments from proteins which have been endocytosed by the APC on which it is expressed, and is recognised by CD4⁺ T-cells (helper T-cells). If a naive CD4⁺ T-cell recognises a peptide presented by a Class II MHC as an antigen, it will proliferate. Its daughter cells will then differentiate into effector, memory and regulatory T-cells, which together mediate an immune response by other components of the immune system, and provide long-term immunity to an infection. Thus, Class I MHCs are generally important in initiating an immune response to virus-infected cells or cells containing mutations causing them to produce abnormal proteins (such as cancerous or pre-cancerous cells); Class II MHCs are generally important in initiating an immune response to extracellular pathogens.

In humans, MHCs consist of proteins known as Human Leukocyte Antigens (HLAs). Every human has 3 main Class I MHC HLA genes (*HLA-A, HLA-B* and *HLA-C*) and 6 main Class II MHC HLA genes (*HLA-DPA1, HLA-DPB1, HLA-DQA1, HLA-DQB1, HLA-DRA,* and *HLA-DR81).* When a TCR binds an MHC-antigen complex, both the antigen and MHC proteins are contacted by the TCR, meaning that TCRs recognise specific MHC-antigen complexes, rather than simply an antigen. This interaction of a TCR with the MHC is believed to be via CDR2, and means that TCRs recognise antigens only when they are complexed with a specific HLA protein, a feature known as MHC restriction. HLA genes are highly polymorphic, meaning that different individuals tend to carry different HLA alleles, and that a specific TCR would not be functional in all individuals (only in those carrying the appropriate HLA allele to which the TCR is restricted).

TCRs which recognise tumour antigens can be used in cancer therapy, specifically in adoptive T-cell transfer therapy (June, C., J Clin Invest. 2007 Jun 1; 117(6): 1466-1476). T-cells can be retargeted against tumour cells by the transfer of genes encoding TCRs which recognise tumour antigens. These re-targeted T-cells can be introduced into a cancer patient suffering from a cancer which produces the relevant antigen. These T-cells should then launch an immune response against the cancerous cells, causing them to be killed. This is hoped to reduce the size of a target tumour, which may result in the patient being cured, or at least their life being extended.

However, recent clinical trials have shown that the adoptive transfer of TCR redirected T-cells targeting cancer germline antigens can be associated with severe toxicity, emphasizing the need for careful consideration of the choice of antigen. In one study, three out of nine cancer patients treated with autologous anti-MAGE-A3 TCR-engineered T-cells (MAGE-A3 being Melanoma-Associated Antigen 3, a protein of unknown function associated with cancers including melanoma but which is also present in healthy cells) experienced severe neurological toxicity (which was lethal in two cases) due to cross-reactivity of the TCR (Morgan, R.A. et al. (2013), Journal of Immunotherapy, 36(2):133-151). A second study targeting MAGE-A3 in myeloma and melanoma patients with a HLA-A*01 restricted TCR demonstrated lethal cross-reactivity with myocardial damage (Linette, G.P. et al. (2013), Blood 122(6):863-871; Cameron, B.J. et al. (2013), Science Translational Medicine 5(197):197ra103). True tumour-specific neoantigens may therefore be the ideal targets for TCR therapy, targeting tumours selectively in the absence of normal tissue destruction. This, however, may be problematic, as the majority of such neoantigens are due to unique mutations not shared between patients. A further issue with adoptive cell transfer therapy with T-cells is that TCRs are, as described above, MHC-limited. An individual TCR, therefore, is only functional in individuals carrying the HLA allele to which it is limited, or a related isoform.

The inventors of the present application have isolated a TCR which has use in adoptive T-cell transfer therapy. This TCR is an HLA-A2 restricted TGFβRII frameshift mutation-specific TCR, isolated from an MSI+ colon cancer patient vaccinated with a TGFβRII frameshift peptide. This TCR, known as Radium-1, has been shown to be particularly effective in re-directing T-cells to recognise cancer cells harbouring the frameshift mutation and reducing cancer growth in an animal model. Radium-1 is an unusually effective TCR with properties which render it particularly useful in medicine. Radium-1 has very high affinity for its cognate antigen/MHC complex. Its affinity for its cognate antigen/MHC complex is higher than that of the MART-1-specific TCR DMF5 for its cognate antigen/MHC complex. DMF5 has been successfully used clinically in melanoma treatment. A high affinity for its antigen is an essential characteristic of a TCR for clinical use, and this high affinity of Radium-1 for its target, in combination with highly successful treatment of tumours in animal models, demonstrates its strong potential for clinical use. Radium-1 also has the unusual property of being CD4 and CD8 co-receptor independent. Most TCRs require interactions between the co-receptors CD8 or CD4 and the MHC complex on a target cell to mediate target cell killing, but this is not the case for Radium-1, meaning that both CD8+ and CD4+ T-cells can functionally express Radium-1 and have been shown to be able to directly mediate target cell killing.

The Radium-1 TCR was isolated from a patient vaccinated with a peptide of SEQ ID NO: 49 (SLVRLSSCVPVALMSAMTTSSSQ). The use of such peptides in human vaccination against cancer is described in WO 1999/058552. Radium-1 was isolated from a T-cell clone obtained from a patient who was vaccinated in the same way as those in the study described in WO 1999/058552. However, the specific patient whence Radium-1 was derived was not a part of that particular study, instead being part of a later clinical trial which took place in 2001. Radium-1 recognises an epitope with the sequence of SEQ ID NO: 1 (RLSSCVPVA). This is a neopeptide resulting from the above-described -1A frameshift mutation of TGFβRII, which as such is an optimal target for adoptive T-cell transfer therapy, as the sequence is not found in the normal human proteome, meaning that TCR toxicity should be minimal. A neoantigen obtained from a frequently occurring frameshift mutation, such as that of SEQ ID NO: 1, is an ideal target for adoptive T-cell transfer therapy.

Furthermore, Radium-1 is HLA-A2 restricted (HLA-A2 is an HLA-A allele). Radium-1 has been demonstrated to recognise antigens in the context of the HLA-A*02:01 isoform of HLA-A2, but may recognise other HLA-A2 isoforms. HLA-A2 is one of the most common HLA alleles, with HLA-A*02:01 being carried by approximately 40 to 50 % of Caucasian Americans and Europeans (www.allelefrequencies.net). The TCR is expected, therefore, to be functional in a significant proportion of the Western population. T-cells re-directed with Radium-1, or a related TCR of the invention, therefore have use in cancer therapy. Particularly, they have use in adoptive cell transfer therapy, e.g. with T-cells. Such re-directed T-cells, or other immune effector cells, have particular use in therapy for any cancer which contains the -1A TGFβRII frameshift mutation, including MSI+ CRCs such as those often seen in sufferers of Lynch Syndrome.

Neopeptides resulting from the -1A frameshift mutation of TGFβRII have previously been described as possible targets for cancer immunotherapy (see e.g. WO 1999/058552; Saeterdal, I. et al., 2001, Proc. Natl. Acad. Sci. USA, Vol. 98, pp. 13255-13260; Saeterdal, I. etal., 2001, Cancer Immunol Immunother 50(9):469-476; Linnebacher, M. et al., 2001, International journal of cancer. Journal international du cancer 93(1):6-11). However, it is not possible to design a functional TCR which will bind a target antigen simply from knowledge of the relevant antigen sequence. It is not possible to predict what protein sequence a TCR would require in order to bind a particular antigen in the context of a particular HLA protein allele. Therefore, despite any recognition that a TCR which binds a neoantigen obtained from a TGFβRII frameshift mutation in the context of a common HLA allele may be useful, the lack of availability of an effective such TCR, and in particular the lack of a known sequence for such a TCR has been a problem, as with the tools available today it is not possible for the skilled man to design such a TCR. It is important to note that not all TCRs recognising a frameshift neoantigen peptide may be effective in practice in stimulating a cell response against a cancer cell expressing the peptide. The isolation and characterisation of the Radium-1 TCR, disclosed herein, and which is just such a TCR, offers a solution to this problem.

The Radium-1 TCR disclosed herein has not previously been publically available. The CDR3 sequences and some information regarding the V and J segments of the α- and β-chains of the Radium-1 TCR were disclosed in the 2011 PhD thesis "Cancer Vaccines and Cancer-Specific T-Cell Therapies; Development of Novel Cancer Immunotherapies" by Else Marit Inderberg Suso, University of Oslo. The present invention now provides full sequence information for the Radium-1 TCR.

Accordingly, in a first aspect the invention provides a nucleic acid molecule encoding a TCR molecule directed against a mutated TGFPRII protein which comprises the sequence of SEQ ID NO: 1, wherein said TCR molecule is capable of binding a peptide of SEQ ID NO: 1 when said peptide is presented by a Class I MHC comprising HLA-A2, wherein said TCR molecule, when expressed by an immune effector cell, is located on the surface of the cell, and wherein said TCR molecule comprises an α-chain domain and a β-chain domain,
wherein said α-chain domain comprises:
   i) a variable region comprising the amino acid sequence set forth in SEQ ID NO: 8; and
   ii) a constant region comprising the amino acid sequence set forth in SEQ ID NO: 9, or a murinised version of SEQ ID NO: 9; and
said β-chain domain comprises:
   i) a variable region comprising the amino acid sequence set forth in SEQ ID NO: 13; and
   ii) a constant region comprising the amino acid sequence set forth in SEQ ID NO: 14, or a murinised version of SEQ ID NO: 14.

The amino acid sequences of CDR1 and CDR2 of the Radium-1 α-chain are presented in SEQ ID NO: 2 and SEQ ID NO: 3 respectively, and the previously disclosed sequence of CDR3 of the Radium-1 α-chain in SEQ ID NO: 4. The amino acid sequences of CDR1 and CDR2 of the Radium-1 β-chain are presented in SEQ ID NO: 5 and SEQ ID NO: 6 respectively, and the previously disclosed sequence of CDR3 of the Radium-1 β-chain in SEQ ID NO: 7.

The CDR sequences of the α-chain are, or correspond to, the CDR sequences of the variable region of the Radium-1 α-chain. The sequence of the variable region of the Radium-1 α-chain is presented in SEQ ID NO: 8. The CDR sequences of the β-chain are, or correspond to, the CDR sequences of the variable region of the Radium-1 β-chain. The sequence of the variable region of the Radium-1 β-chain is presented in SEQ ID NO: 13. SEQ ID NOs: 2, 3 and 4, corresponding respectively to CDRs 1, 2 and 3 of the Radium-1 α-chain, are located at positions 47-51, 69-73 and 107-117 of SEQ ID NO: 8, respectively. SEQ ID NOs: 5, 6 and 7, corresponding respectively to CDRs 1, 2 and 3 of the Radium-1 β-chain, are located at positions 46-50, 68-73 and 110-122 of SEQ ID NO: 13, respectively.

The nucleic acid molecule of the first aspect of the invention may be used to prepare immune effector cells (more particularly modified immune effector cells) directed against cells expressing a mutated TGFβRII receptor, or more particularly presenting the frameshift peptide of SEQ ID NO: 1. Such (modified) immune effector cells express the TCR on their cell surface and are capable of recognising, or binding to, a target cell presenting the peptide of SEQ ID NO: 1, e.g. a cancer cell. Accordingly, the nucleic acid molecule is such that an immune effector cell expressing said TCR (i.e. the TCR encoded by the nucleic acid molecule) is capable of effector activity (e.g. cytotoxic activity) against (e.g. killing) a target cell presenting the frameshift peptide of SEQ ID NO: 1. In other words, the nucleic acid molecule encodes a TCR molecule which, when expressed on the surface of an immune effector cell, is capable of binding a peptide of SEQ ID NO: 1 when said peptide is presented by a Class I MHC comprising HLA-A2. A modified immune effector cell is accordingly a genetically modified or engineered immune effector cell, or alternatively expressed an immune effector cell which has been transduced with a nucleic acid molecule of the first aspect of the invention.

The nucleic acid molecule of the invention may alternatively be used for expression of a soluble TCR molecule by a host cell. Thus in a second aspect the invention provides a nucleic acid molecule encoding a soluble TCR molecule directed against a mutated TGFβRII protein which comprises the sequence of SEQ ID NO: 1, wherein said TCR molecule is capable of binding a peptide of SEQ ID NO: 1 when said peptide is presented by a Class I MHC comprising HLA-A2, and wherein said soluble TCR molecule comprises an α-chain domain and a β-chain domain,
wherein said α-chain domain comprises:
   i) a variable region comprising the amino acid sequence set forth in SEQ ID NO: 72; and
   ii) a constant region comprising the amino acid sequence set forth in SEQ ID NO: 60 or SEQ ID NO: 61; and
said β-chain domain comprises:
   i) a variable region comprising the amino acid sequence set forth in SEQ ID NO: 75; and
   ii) a constant region comprising the amino acid sequence set forth in SEQ ID NO: 62 or SEQ ID NO: 63.

The soluble TCR molecule of the invention is thus capable of binding a peptide of SEQ ID NO: 1 when said peptide is presented by a Class I MHC comprising HLA-A2, and may in particular be used to deliver a toxin to a target cell presenting the frameshift peptide of SEQ ID NO: 1, in order to kill the target cell.

The nucleic acid molecule of the first or second aspect of the invention may be introduced into a cell, notably an immune effector cell such as a T-cell or a production host cell, as mRNA or as DNA for expression in the cell. Vectors may be used to transfer the nucleic acid molecule into the cell or to produce the nucleic acid for transfer (e.g. to produce mRNA for transfer, or to produce a nucleic acid molecule for preparation of an expression vector for transfer into a cell).

Accordingly, a further aspect of the invention provides a vector comprising a nucleic acid molecule of the invention as defined herein.

The vector may for example be an mRNA expression vector, a cloning vector or an expression vector for transfer into an immune cell or a production host cell, e.g. a viral vector. If the vector is a viral vector, it may for example be a retroviral vector or a lentiviral vector.

Another aspect of the invention provides a host cell comprising a nucleic acid molecule of the invention or a vector of the invention.

The host cell may be an immune effector cell comprising a nucleic acid molecule of the first aspect of the invention or a vector of the invention comprising such a nucleic acid molecule, and expressing a TCR as defined in the first aspect of the invention. In preferred embodiments the immune effector cell may be a T-cell or an NK cell.

In a particular embodiment wherein the immune effector cell is a T-cell (or more particularly a CD8⁺ T-cell or a human CD8⁺ T-cell), the TCR, or more particularly the nucleic acid molecule, is not native to the T-cell, i.e. the nucleic acid molecule is not endogenously present in the T-cell but is introduced into the T-cell. In other words the T-cell is modified with the nucleic acid molecule or vector, i.e. it is modified to express the TCR; it is not a native, or naturally-occurring T-cell.

The host cell may alternatively be a production host cell comprising a nucleic acid molecule of the second aspect of the invention or a vector of the invention comprising such a nucleic acid molecule, as defined herein. In preferred embodiments the production host is a mammalian cell, e.g. a HEK-293, HEK-293T or CHO cell.

Also provided is an *in vitro* or *ex vivo* method of generating a TGFβRII frameshift mutant-specific immune effector cell, said method comprising introducing into an immune effector cell a nucleic acid molecule of the first aspect of the invention or a vector of the invention comprising such a nucleic acid molecule.

Such a method may comprise stimulating the cell and inducing it to proliferate before and/or after introducing the nucleic acid molecule or vector.

The invention also provides a TCR molecule as defined herein, in particular a soluble TCR molecule as defined herein. As described above and further described below, soluble TCRs have utility in therapy.

As noted above, soluble TCRs and immune effector cells of the invention have a utility in therapy. Accordingly, further aspects of the invention include:
a composition, particularly a therapeutic or pharmaceutical composition, comprising a soluble TCR or an immune effector cell of the invention as defined herein and at least one physiologically acceptable carrier or excipient;
a soluble TCR, an immune effector cell or a composition of the invention as defined herein for use in therapy, particularly adoptive cell transfer therapy; and
a soluble TCR, an immune effector cell or a composition of the invention as defined herein for use in the treatment of cancer, wherein said cancer expresses a mutated TGFβRII protein which comprises SEQ ID NO: 1; particularly for the treatment of colorectal cancer caused by HNPCC.

In the method of generating a modified immune effector cell of the invention, the immune effector cell which is modified by introduction of the nucleic acid molecule of the invention may be obtained from a subject to be treated (e.g. a subject with a cancer, such as a colorectal cancer). After modification of the immune effector cell, and optionally *in vitro* expansion thereof, the modified immune effector cells expressing the TCR may be reintroduced (i.e. administered) to the subject. Thus, autologous immune effector cells may be used in the therapeutic aspects of the invention. Alternatively, heterologous (i.e. donor or allogeneic, or syngeneic or xenogeneic) immune effector cells may be used.

An immune effector cell may be any immune cell capable of an immune response against a target cell presenting the peptide of SEQ ID NO: 1. More particularly, the immune effector cell is capable of abrogating, damaging or deleting a target cell, i.e. of reducing, or inhibiting, the viability of a target cell, preferably killing a target cell (in other words rendering a target cell less or non-viable). The immune effector cell is thus preferably a cytotoxic immune effector cell.

The term "cytotoxic" is synonymous with "cytolytic" and is used herein to refer to a cell capable of inducing cell death by lysis or apoptosis in a target cell.

The term "immune effector cell" as used herein includes not only mature or fully differentiated immune effector cells but also precursor (or progenitor) cells therefor, including stem cells (more particularly haemopoietic stem cells, HSC), or cells derived from HSC. An immune effector cell may accordingly be a T-cell, NK cell, NKT cell, neutrophil, macrophage, or a cell derived from HSCs contained within the CD34+ population of cells derived from a haemopoietic tissue, e.g. from bone marrow, cord blood, or blood e.g. mobilised peripheral blood, which upon administration to a subject differentiate into mature immune effector cells. As will be described in more detail below, in preferred embodiments, the immune effector cell is a T-cell or an NK cell. Primary cells, e.g. cells isolated from a subject to be treated or from a donor subject may be used, optionally with an intervening cell culture step (e.g. to expand the cells) or other cultured cells or cell lines (e.g. NK cell lines such as the NK92 cell line).

The term "directed against the peptide of SEQ ID NO: 1" is synonymous with "specific for the peptide of SEQ ID NO: 1", that is it means simply that the TCR is capable of binding specifically to the peptide. In particular, the antigen-binding domain of the TCR is capable of binding specifically to the peptide (more particularly when the TCR is expressed on the surface of an immune effector cell). Specific binding may be distinguished from non-specific binding to a non-target antigen (in this case a peptide other than the peptide of SEQ ID NO: 1). Thus, an immune effector cell expressing the TCR according to the present invention is redirected to bind specifically to and exhibit cytotoxicity to (e.g. kill) a target cell presenting the peptide of SEQ ID NO: 1. Alternatively expressed, the immune effector cell is modified to redirect cytotoxicity towards target cells presenting the peptide, or expressing a mutant TGFβRII receptor comprising the peptide.

The binding of the antigen binding domain of the TCR to the peptide on the surface of the target cell delivers an activation stimulus to the TCR-containing cell, resulting in induction of effector cell signalling pathways. Binding to target peptide may thereby trigger proliferation, cytokine production, phagocytosis, lytic activity and/or production of molecules that can mediate cell death of the target cell in an MHC-independent manner.

The soluble TCR of the invention may be produced by any suitable production host cell. Such a cell is preferably a mammalian cell, for instance a human cell or a rodent cell. Any suitable cell line may be used, including HEK-293, HEK-293T and CHO cells. Binding of a soluble TCR of the invention to the peptide on the surface of the target cell leads to internalisation of the MHC Class I-antigen-TCR complex. Soluble TCRs may thus be used to specifically deliver toxins to target cells, resulting in target cell death.

The α-chain domain and the β-chain domain of the TCR of the invention may be encoded separately (i.e. may be encoded by separate genes, or more particularly by separate nucleic acid molecules or separate parts (in the sense of separately controlled parts) or open reading frames (ORFs) of the nucleic acid molecule and synthesised as separate proteins). Alternatively, they may be encoded together, by a single gene (i.e. a single nucleic acid molecule, or single ORF etc.), in which case they are synthesised as a single protein. In the case that the α-chain domain and the β-chain domain are encoded as a single protein, this protein is known as a single-chain TCR (scTCR). A scTCR comprises an α-chain domain linked to a β-chain domain. The term "a-chain domain", as used herein, refers to a TCR α-chain which either constitutes an individual protein or which forms part of a protein, and particularly part of a scTCR. The term "β-chain domain", as used herein, refers to a TCR β-chain which either constitutes an individual protein or which forms part of a protein, and particularly part of a scTCR. Expression of the α- and β-chain domains in a single scTCR molecule ensures that the two chain domains are expressed at the same time and at similar levels. In a preferred embodiment of the invention, the TCR molecule of the invention is encoded as a scTCR.

In the case that the TCR molecule of the invention is encoded as a scTCR, the α- and β-chain domains may be joined by a linker. This linker consists of an amino acid sequence between the α- and β-chain domains. Preferably, the α-chain domain is at the N-terminus of the scTCR, followed by the linker, followed by the β-chain domain at the C-terminus of the scTCR. However, the β-chain domain could alternatively be located at the N-terminus of the scTCR with the α-chain domain at the C-terminus, and the linker in-between.

The amino acid sequence of the linker can be of any suitable length. The linker sequence may be 1-30 amino acids long, or more preferably 1-25 or 1-20 amino acids long. However, the linker should, preferably, be cleavable, such that the two TCR chains can be separated; unless they can be separated, the two chains may not be able to adopt the correct conformations and interact properly, which could lead to the TCR being non-functional.

In a preferred embodiment, the linker is self-splicing. A self-splicing linker is able to catalyse cleavage of the scTCR molecule at the position of the linker, thus separating the α- and β-chain domains. No stimulation or induction is required for this splicing reaction to occur, and the splicing reaction ideally occurs prior to the transport of the α- and β-chain domains to the cell surface. The cleavage reaction may completely excise the linker from the TCR molecule; alternatively the linker, or a part of the linker, may remain attached to one or both resultant separate TCR chains. The splicing reaction catalysed by the linker may occur post-translationally (i.e. it may be an autocatalytic proteolysis reaction), or it may occur co-translationally. Co-translational splicing can occur by preventing the formation of a peptide bond within the linker or between the linker and one of the chain domains on either side of it.

A preferred self-splicing linker is one derived from a picornavirus self-cleaving 2A peptide. 2A peptides are approximately 20-25 amino acids long and end with the conserved sequence motif Asp-Val/lle-Glu-X-Asn-Pro-Gly-Pro (SEQ ID NO: 52). 2A peptides undergo co-translational self-splicing, by preventing the formation of a peptide bond between the conserved glycine reside and the final proline residue, resulting effectively in cleavage of the protein between these two amino acids. After cleavage, the 2A peptide (with the exception of the C-terminal proline) remains attached to the C-terminus of the upstream protein; the final proline residue remains attached to the N-terminus of the downstream protein. A particularly preferred sequence of a 2A peptide-derived linker is presented in SEQ ID NO: 18. However, the sequence of the peptide upstream of the conserved C-terminal 2A sequence motif (SEQ ID NO: 52) may be varied without significant loss of self-splicing activity. The linker may thus also have a sequence with at least 40 %, 45 %, 50 %, 55 %, 60 %, 65 %, 70 %, 75 %, 80 %, 85 %, 90 % or 95 % sequence identity to that of SEQ ID NO: 18, so long as it ends with the above-described conserved sequence motif and retains self-splicing activity. In particular, self-splicing variants of SEQ ID NO: 18 may retain at least 70 %, 75 %, 80 %, 85 %, 90 % or 95 % of the self-splicing activity of the peptide of SEQ ID NO: 18.

A full-length TCR of the invention (i.e. a non-soluble TCR which, when expressed by an immune effector cell, localises to the cell surface), when expressed on the surface of an immune effector cell, such as a T-cell, is capable of re-directing the cell on which it is expressed such that the cell recognises the neo-antigen of SEQ ID NO: 1 when it is presented by a Class I MHC comprising HLA-A2. In other words, such a TCR of the invention activates an immune effector cell to direct its effect or function, e.g. its cytotoxic activity, against a target cell which has suffered a -1A frameshift mutation in *TGFβRII,* or indeed any similar mutation which results in the generation of the neo-antigen with SEQ ID NO: 1. The immune effector cell, upon whose surface the full-length TCR of the invention is expressed, may be any immune effector cell as discussed above and further below, but in one preferred embodiment it is a CD4⁺ T-cell, a CD8⁺ T-cell, or any other type of T-cell.

A soluble TCR of the invention is capable of recognising, i.e. binding, the neo-antigen of SEQ ID NO: 1 when it is presented by a Class I MHC comprising HLA-A2, and is thus selectively internalised by a target cell. Selective internalisation of soluble TCRs of the invention may be identified by any method known in the art. For instance, the soluble TCR may be conjugated to a fluorophore (e.g. a fluorescent protein such as GFP), and internalisation of the TCR thus identified by internalisation of fluorescence. If the soluble TCR is internalised by cells which express the neo-antigen of SEQ ID NO:1, but is not internalised (or is internalised to a lower degree) by cells which do not express SEQ ID NO:1, the soluble TCR can be said to be selectively internalised by target cells. As described above, soluble TCRs comprise both an α- and a β-chain, but each chain is truncated at its C-terminus by the deletion of the transmembrane and intracellular domains of the constant region. The chains of a soluble TCR thus comprise an N-terminal leader sequence (until it is cleaved during maturation of the polypeptides), a variable region and the N-terminus (i.e. the extracellular domain) of the constant region. A soluble TCR can thus be said to be a truncated TCR, with truncated α- and β-chains, while an insoluble TCR (such as a wild-type TCR), which is expressed on the surface of an immune effector cell, such as a T-cell, can be said to be a full-length TCR, with full-length α- and β-chains.

As mentioned above, the Class I MHC which presents the neo-antigen of SEQ ID NO: 1 to the TCR of the invention (the TCR either being in the context of a solution, i.e. a soluble TCR, or a T-cell expressing the full-length TCR of the invention) comprises the HLA-A allele HLA-A2. More specifically, the Class I MHC may comprise the HLA-A*02:01 isoform of HLA-A2. However, the Class I MHC is not limited to those comprising HLA-A*02:01. In particular, it may comprise an HLA-A2 isoform other than HLA-A*02:01. In other words it may comprise any isoform of HLA-A2.

As mentioned above, the CDRs of a TCR α- or β-chain are located within the variable region of the chain. Each variable region comprises 3 CDR sequences in a scaffold of 4 framework sequences. It would not be possible, merely from the CDR sequences, to predict what framework region sequences would hold the CDR sequences together in a functional TCR. As mentioned, the amino acid sequences of the variable regions of the α- and β-chains of the Radium-1 TCR are presented in SEQ ID NOs: 8 and 13, respectively. In the TCR of the invention the framework regions of the variable regions of the α- and/or β-chain domains are the same as the framework regions of the native Radium-1 receptor (i.e. as it was isolated, or found in nature).

The α-chain domain of the full-length TCR comprises a variable region with, or comprising or consisting of, the amino acid sequence of SEQ ID NO: 8, and the β-chain domain of the full-length TCR comprises a variable region with, or comprising or consisting of, the amino acid sequence of SEQ ID NO: 13.

As discussed above, adoptive cell transfer therapy can pose safety risks to patients. As a failsafe mechanism, it is possible to encode a tag within the TCR of the invention which allows targeted killing of cells expressing the full-length TCR. Such targeted killing can then be performed if the patient suffers a negative reaction to the therapy. The targeted cell-killing can be performed using antibodies which recognise the introduced tag. Such a mechanism is described in Kieback, E. et al., 2007, Proc. Natl. Acad. Sci. USA, Vol. 105, pp. 623-628. In one embodiment of the invention, the TCR comprises a common tag sequence. Examples of such a tag are well known in the art, and include a FLAG-tag, a polyhistidine-tag (His-tag), an HA-tag, a Strep-tag, an S-tag and a Myc-tag. In a preferred embodiment the TCR of the invention comprises a Myc-tag. Multiple (i.e. two or more, e.g. 2 to 10, 2 to 8 or 2 to 6), preferably contiguous, copies of the tag sequence may be present in the TCR. In a particularly preferred embodiment, the TCR comprises a double Myc-tag. Such a double Myc-tag has the amino acid sequence presented in SEQ ID NO: 19.

The tag may be located in either chain of the TCR. In one embodiment, the α-chain domain comprises a variable region which further comprises a double Myc-tag with the amino acid sequence of SEQ ID NO: 19. In another embodiment, the β-chain domain comprises a variable region which further comprises a double Myc-tag with the amino acid sequence of SEQ ID NO: 19. In a further embodiment both the α- and β-chain domains comprise variable regions which comprise a double Myc-tag.

As mentioned above, the N-terminus of a TCR chain as synthesised constitutes a signal peptide. Such a signal peptide is generally between about 15 and about 30 amino acids in length. The signal peptide for the Radium-1 α-chain is predicted to consist of the first 20 amino acids of SEQ ID NO: 8, represented by SEQ ID NO: 50. The signal peptide for the Radium-1 β-chain is predicted to consist of the first 16 amino acids of SEQ ID NO: 13, represented by SEQ ID NO: 51. As mentioned above, these leader sequences are not present in the mature TCR.

As described above, both α- and β-TCR chains comprise a variable and a constant region. The sequence of the constant region of the α-chain of the Radium-1 TCR is presented in SEQ ID NO: 9, and the sequence of the constant region of the β-chain of the Radium-1 TCR is presented in SEQ ID NO: 14. Like all full-length TCR chain constant regions, these comprise an extracellular domain, a transmembrane helix and a short intracellular domain (as previously mentioned, the constant regions of the truncated TCR chains of a soluble TCR comprise only the extracellular domain of the corresponding full-length sequence).

The constant region of the α-chain domain of the full-length TCR of the invention may have, or comprise or consist of, the sequence of the constant region of the Radium-1 α-chain. In other words, the α-chain domain may comprise a constant region with, i.e. comprising or consisting of, the sequence of SEQ ID NO: 9.

Alternatively, the α-chain domain of the full-length TCR of the invention may comprise a constant region which has, or comprises or consists of, the sequence of a murine equivalent to the constant region of the Radium-1 α-chain. That is to say, the α-chain domain may comprise a constant region with a sequence which is a murinised version of SEQ ID NO: 9. Such an α-chain domain could be seen to have had its human constant region exchanged for a murine constant region.

The sequence of a murine TCR α-chain constant domain which is equivalent to that of the Radium-1 TCR α-chain constant domain presented in SEQ ID NO: 9 is that of SEQ ID NO: 23. In a particular embodiment in which the constant region of the α-chain domain is murinised, the murinised constant region has, or comprises or consists of, the amino acid sequence of SEQ ID NO: 23.

The constant region of the β-chain domain of the full-length TCR of the invention may have, or comprise or consist of, the sequence of the constant region of the Radium-1 β-chain. In other words, the β-chain domain may comprise a constant region with, i.e. comprising or consisting of, the sequence of SEQ ID NO: 14.

Alternatively, the β-chain domain of the full-length TCR of the invention may comprise a constant region which has, or comprises or consists of, the sequence of a murine equivalent to the constant region of the Radium-1 β-chain. That is to say, the β-chain domain may comprise a constant region with a sequence which is a murinised version of SEQ ID NO: 14. Such a β-chain domain could be seen to have had its human constant region exchanged for a murine constant region.

The sequence of a murine TCR β-chain constant domain which is equivalent to that of the Radium-1 TCR β-chain constant domain presented in SEQ ID NO: 14 is that of SEQ ID NO: 29. In a particular embodiment in which the constant region of the β-chain domain is murinised, the murinised constant region has, or comprises or consists of, the amino acid sequence of SEQ ID NO: 29.

In the TCR of the invention, though the α- and β-chains can be encoded as a single polypeptide chain (i.e. as a scTCR), in their mature forms they form separate chains. Either the α- and β-chains are encoded as separate polypeptides, or they are encoded as a scTCR, in which case they are joined by a linker which is cleaved prior to their maturation and transport to the cell membrane. This means that in mature TCRs of the invention the α- and β- chains form discrete polypeptide chains: i.e. they are no longer joined by peptide bonds.

In all mature αβ-TCRs the α- and β-chains are covalently joined by inter-chain disulphide bonds, which form between cysteine residues located in the constant regions of each chain. The inter-chain disulphide bonds ensure that the two TCR chains remain in close association once the TCR is formed, which is essential for TCR functionality.

As herein described, the α- and β-chain domains of the full-length TCR of the invention each comprises or consists of a variable region and a constant region with the variable and constant region sequences defined and described above.

In one embodiment of the full-length TCR of the invention, the α-chain domain comprises a variable region and a constant region with the respective sequences of the variable and constant regions of the α-chain of the Radium-1 TCR. In this embodiment, the α-chain domain of the invention may have the sequence of the α-chain of the Radium-1 TCR, which is presented in SEQ ID NO: 11. Therefore, in one embodiment of the invention, the α-chain domain has, or comprises or consists of, the amino acid sequence of SEQ ID NO: 11.

As described above, the constant region of the α-chain domain of the invention may alternatively be a murine (or murinised) version of SEQ ID NO: 9. In preferred embodiments of the invention, when the constant region of the full-length TCR α-chain domain is murine it has the sequence of SEQ ID NO: 23. The sequence of a full-length TCR α-chain with the variable region of the Radium-1 α-chain and a constant region with the sequence of SEQ ID NO: 23 is presented in SEQ ID NO: 25. In an embodiment of the invention, the α-chain domain of the TCR comprises a murinised constant region and a human variable region, and has, or comprises or consists of, the sequence of SEQ ID NO: 25.

Similarly to the α-chain domain, in one embodiment of the invention, the full-length β-chain domain comprises a variable region and a constant region with the respective sequences of the variable and constant regions of the β-chain of the Radium-1 TCR. In this embodiment, the β-chain domain of the invention may have the sequence of the β-chain of the Radium-1 TCR, which is presented in SEQ ID NO: 16. Therefore, in one embodiment of the invention, the β-chain domain has, or comprises or consists of, the amino acid sequence of SEQ ID NO: 16.

As for the α-chain domain, the constant region of the full-length β-chain domain of the invention may alternatively be a murine (or murinised) version of SEQ ID NO: 14. In preferred embodiments of the invention, when the constant region of the full-length TCR β-chain domain is murine it has the sequence of SEQ ID NO: 29, while the variable region is that of the Radium-1 β-chain. The sequence of a TCR β-chain with the variable region of the Radium-1 β-chain and a constant region with the sequence of SEQ ID NO: 29 is presented in SEQ ID NO: 31. In an embodiment of the invention, the β-chain domain of the TCR comprises a murinised constant region and a human variable region, and has, or comprises or consists of, the sequence of SEQ ID NO: 31.

In the soluble TCR of the invention, the constant regions of the α- and β-chain domains may correspond to truncated versions of the full-length constant regions described above. In a particular embodiment of the invention, the truncated constant region of the α-chain domain corresponds to amino acids 1-95 of the constant region of the Radium-1 α-chain (i.e. amino acids 1-95 of SEQ ID NO: 9). This sequence is set forth in SEQ ID NO: 60. The soluble TCR of the invention may comprise an α-chain domain comprising a constant region comprising or consisting of the amino acid sequence set forth in SEQ ID NO: 60. In another embodiment of the invention, the truncated constant region of the β-chain domain corresponds to amino acids 1-131 of the constant region of the Radium-1 β-chain (i.e. amino acids 1-131 of SEQ ID NO: 14). This sequence is set forth in SEQ ID NO: 62. The soluble TCR of the invention may comprise a β-chain domain comprising a constant region comprising or consisting of the amino acid sequence set forth in SEQ ID NO: 62.

It is an essential aspect of a soluble TCR that the α- and β-chains of the mature TCR are joined. If they are not joined, the chains will diffuse apart in solution and the TCR will function poorly, if at all. The chains may be joined covalently or non-covalently. A preferred method by which the α- and β-chains can be covalently joined is by one or more disulphide bonds. These may form between cysteine residues present in the native TCR chain sequences, but in a preferred embodiment one or more cysteine residues are introduced into the constant regions of each chain, between which disulphide bonds can form. TCR chains or chain domains of the invention into which an extra cysteine has been introduced may be referred to as "cysteine-modified". In a preferred embodiment of the invention, the constant regions of the α- and β-chain domains of the soluble TCR are cysteine-modified. Such an extra cysteine residue may be introduced by insertion (i.e. by inserting an extra amino acid residue into the constant region of the α- or β-chain domain) or by substitution (i.e. by substituting a non-cysteine amino acid already present in the constant region of the α- or β-chain domain for cysteine). If a cysteine residue is to be introduced into the constant regions of both the α- and β-chain domains, different methods of cysteine introduction may be used in each chain domain.

In a particularly preferred embodiment, the truncated constant region of the Radium-1 α-chain (i.e. SEQ ID NO: 60) is modified by the T48C substitution. The sequence of such a cysteine-modified truncated α-chain domain constant region is set forth in SEQ ID NO: 61 (and corresponds to amino acids 1-95 of SEQ ID NO: 10, which sets forth the cysteine-modified sequence of the full-length Radium-1 α-chain constant region). Thus in a preferred embodiment, the soluble TCR of the invention comprises an α-chain domain comprising a constant region comprising or consisting of the amino acid sequence set forth in SEQ ID NO: 61.

In another preferred embodiment, the truncated constant region of the Radium-1 β-chain (i.e. SEQ ID NO: 62) is modified by the S57C substitution. The sequence of such a cysteine-modified truncated β-chain domain constant region is set forth in SEQ ID NO: 63 (and corresponds to amino acids 1-131 of SEQ ID NO: 15, which sets forth the cysteine-modified sequence of the full-length Radium-1 β-chain constant region). Thus in a preferred embodiment, the soluble TCR of the invention comprises a β-chain domain comprising a constant region comprising or consisting of the amino acid sequence set forth in SEQ ID NO: 63.

An alternative method by which the α- and β-chains of the soluble TCR may be joined is by non-covalent interactions. In a particular embodiment, leucine zippers are used to non-covalently join the chains. In this embodiment, both the α- and β-chains comprise leucine zipper domains at the C-terminus of their truncated constant regions (i.e. the α-chain comprises a leucine zipper domain at its C-terminus and the β-chain comprises a leucine zipper domain at its C-terminus). Leucine zippers, and their sequences, are well-known in the art, and are reviewed in e.g. Busch & Sassone-Corsi (1990), Trends Genet 6: 36-40. In some embodiments, both covalent and non-covalent methods may be used to join the α- and β-chains of the soluble TCR, e.g. the α- and β-chains may be both cysteine-modified and include leucine zipper domains.

The soluble TCR of the invention may thus comprise an α-chain domain corresponding to a truncated α-chain of Radium-1, in which the C-terminal 46 amino acids are absent. Such a truncated α-chain has the sequence set forth in SEQ ID NO: 64 (corresponding to residues 1-222 of SEQ ID NO: 11). If the constant region is cysteine-modified as described above (i.e. the constant region contains a Thr → Cys substitution relative to the wild-type sequence, corresponding to a T175C substitution in SEQ ID

NO: 64), the truncated α-chain has the sequence set forth in SEQ ID NO: 65. In a preferred embodiment, the α-chain domain of the soluble TCR comprises or consists of the sequence set forth in SEQ ID NO: 64 or SEQ ID NO: 65.

The β-chain domain of the soluble TCR of the invention may correspond to a truncated β-chain of Radium-1, in which the C-terminal 48 amino acids are absent. Such a truncated β-chain has the sequence set forth in SEQ ID NO: 66 (corresponding to residues 1-262 of SEQ ID NO: 16). If the constant region is cysteine-modified as described above (i.e. the constant region contains a Ser → Cys substitution relative to the wild-type sequence, corresponding to an S188C substitution in SEQ ID NO:66), the truncated β-chain has the sequence set forth in SEQ ID NO: 67. In a preferred embodiment, the β-chain domain of the soluble TCR comprises or consists of the sequence set forth in SEQ ID NO: 66 or SEQ ID NO: 67.

In certain embodiments, a purification tag as described above is encoded at the C-terminus of the α- or β-chain domain. A preferred tag is a hexahistidine (His-) tag. Such a tag may be joined to the C-terminus of the α- or β-chain by a linker, such as the short linker Gly-Gly-Gly.

The TCR of the invention may comprise any combination of the above-described α- and β-chain domains of the invention. For instance, an α-chain domain comprising a human constant region may be paired with a β-chain comprising a murine constant region, and vice-versa. It is generally preferred though that like should be paired with like, such that, for instance, an α-chain domain comprising a human constant region is paired with a β-chain domain comprising a human constant region, and vice-versa; an α-chain domain comprising a murine constant region is paired with a β-chain domain comprising a murine constant region, and vice versa; or an α-chain domain comprising a constant region which has been cysteine-modified is paired with a β-chain domain comprising a constant region which has been cysteine-modified, and vice-versa.

As described above, in preferred embodiments of the invention, the TCR is encoded as an scTCR, wherein the C-terminus of the α-chain domain is joined to the N-terminus of the β-chain domain by a linker. The linker should be cleavable: preferably it is a self-splicing linker, such as linker derived from the picornavirus 2A peptide. Most preferably it has the sequence of SEQ ID NO: 18, or a variant thereof.

A full-length TCR of the invention may be encoded as an scTCR. In one such embodiment, the scTCR comprises an α-chain domain with the sequence of the Radium-1 α-chain and a β-chain domain with the sequence of the Radium-1 β-chain, joined by the linker of SEQ ID NO: 18. Such an scTCR has the amino acid sequence of SEQ ID NO: 33.

In another preferred embodiment, particularly of the full-length TCR of the invention, the variable region of the α- and/or β-chain domain of a TCR of the invention comprises the sequence of a tag, preferably a double Myc-tag. Preferably, the variable region of only one of the α-chain domain or the β-chain domain comprises the sequence of a tag, most preferably the variable region of the α-chain domain.

Thus, in one embodiment of the invention, the TCR molecule is an scTCR with the amino acid sequence of SEQ ID NO: 33.

As discussed above, the constant regions of the α- and/or β-chain domains of the invention may be murinised. A preferred full-length α-chain domain with a murine constant region has the sequence of SEQ ID NO: 25, and a preferred full-length β-chain domain with a murine constant region has the sequence of SEQ ID NO: 31. An scTCR comprising an α-chain domain with the sequence of SEQ ID NO: 25 and a β-chain domain of SEQ ID NO: 31 joined by the linker of SEQ ID NO: 18 has the sequence of SEQ ID NO: 37.

Thus, in a particular embodiment of the invention, the TCR molecule is an scTCR with the amino acid sequence of SEQ ID NO: 37.

The scTCR polypeptides with SEQ ID NOs: 33 and 37 are encoded by the nucleotide sequences of SEQ ID NOs: 41 and 45, respectively. A nucleic acid molecule of the invention is one which encodes a TCR molecule of the invention. A nucleic acid molecule of the invention may therefore comprise a nucleotide sequence which encodes any TCR molecule of the invention as defined above. A nucleic acid molecule of the invention which encodes an scTCR with α- and β-chain domains which comprise human constant regions may in particular comprise the nucleotide sequence of SEQ ID NO: 41. A nucleic acid molecule of the invention which encodes an scTCR with α- and β-chain domains which comprise murine constant regions may in particular comprise the nucleotide sequence of SEQ ID NO: 45.

Thus, a nucleic acid molecule of the invention may comprise the nucleotide sequence of SEQ ID NO: 41 or 45. Alternatively, a nucleic acid molecule of the invention may comprise a nucleotide sequence which is degenerate to the nucleotide sequence of SEQ ID NO: 41 or 45. A nucleic acid molecule which comprises a nucleotide sequence which has a sequence which is the reverse complement to any of the above-defined nucleic acid molecules of the invention also falls under the scope of the invention.

A soluble TCR of the invention may be encoded as an scTCR. Preferred scTCRs of soluble TCRs correspond to those described above for full-length TCRs, using the corresponding truncated α- and β-chain domains. As for the full-length TCR of the invention, it is preferred that the scTCR is encoded with the α-chain domain at its N-terminus and the β-chain domain at its C-terminus, with the two domains separated by a linker, preferably the picornavirus 2A peptide with the sequence set forth in SEQ ID NO: 18, or a derivative thereof, as defined above.

In an embodiment, the soluble scTCR comprises an α-chain domain with the amino acid sequence set forth in SEQ ID NO: 65 and a β-chain domain with the amino acid sequence set forth in SEQ ID NO: 67, the two chains separated by a 2A-derived linker. In a particular embodiment this scTCR comprises or consists of the sequence set forth in SEQ ID NO: 68..

In another embodiment, a His-tag is located at the C-terminus of the scTCR of SEQ ID NO: 68 (the C-terminus of the scTCR corresponding to the C-terminus of the β-chain), the His-tag being separated from the β-chain by a Gly-Gly-Gly linker. Such an scTCR has the sequence set forth in SEQ ID NO: 69. In another preferred embodiment, the invention provides an scTCR comprising or consisting of the sequence set forth in SEQ ID NO: 69.

The amino acid sequences of SEQ ID NOs: 68 and 69 are encoded by the nucleotide sequences set forth in SEQ ID NOs: 70 and 71. A nucleic acid molecule of the invention is a nucleic acid molecule which encodes a TCR of the invention, including nucleic acid molecules which encode full-length TCRs and nucleic acid molecules which encode soluble TCRs. A nucleic acid molecule of the invention is in certain embodiments a nucleic acid molecule which comprises or consists of the nucleotide sequence set forth in SEQ ID NO: 70 or SEQ ID NO: 71. A nucleic acid molecule comprising or consisting of the reverse complement of SEQ ID NO: 70 or SEQ ID NO: 71 also falls within the scope of the invention.

As described above, certain embodiments of the invention refer to polypeptides or polynucleotides with a certain level of sequence identity to a particular, defined sequence (the reference sequence). Where % sequence identity is given herein with respect to a particular reference sequence, the % sequence identity is determined over the whole length of the reference sequence. When comparing polypeptide or polynucleotide sequences, two sequences are said to be "identical" if the sequence of nucleotides in the two sequences is the same when aligned for maximum correspondence. Methods for determining sequence identity are well known in the art and any convenient or available method may be used.

Optimal alignment of sequences for comparison may be conducted using the Megalign program in the Lasergene suite of bioinformatics software (DNASTAR, Inc., Madison, Wl), using default parameters. Alternatively, optimal alignment of sequences for comparison may be conducted by the local identity algorithm of Smith and Waterman, Add. APL. Math 2:482 (1981), by the identity alignment algorithm of Needleman and Wunsch, J. Mol. Biol. 48:443 (1970), by the search for similarity methods of Pearson and Lipman, Proc. Natl. Acad. Sci. USA 85: 2444 (1988), by computerized implementations of these algorithms (GAP, BESTFIT, BLAST, FASTA, and TFASTA in the Wisconsin Genetics Software Package, Genetics Computer Group (GCG), 575 Science Dr., Madison, Wl), or by inspection.

It will be appreciated by those of ordinary skill in the art that, as a result of the degeneracy of the genetic code, there are many nucleotide sequences that may encode each individual TCR molecule as described herein.

The nucleic acid molecule of the invention may be an isolated nucleic acid molecule and may include DNA (including cDNA) or RNA or chemical derivatives of DNA or RNA, including molecules having a radioactive isotope or a chemical adduct such as a fluorophore, chromophore or biotin ("label"). Thus the nucleic acid may comprise modified nucleotides. Said modifications include base modifications such as bromouridine, ribose modifications such as arabinoside and 2',3'-dideoxyribose and internucleotide linkage modifications such as phosphorothioate, phosphorodithioate, phosphoroselenoate, phosphorodiselenoate, phosphoroanilothioate, phosphoraniladate and phosphoroamidate. The term "nucleic acid molecule" specifically includes single and double stranded forms of DNA and RNA.

Methods for modifying nucleotide sequences to introduce changes to the amino acid sequences of the various TCR regions are well known in the art, e.g. methods of mutagenesis, such as site-specific mutagenesis, may be employed. Methods for preparing a nucleic acid molecule encoding the TCR molecule are also well known, e.g. conventional polymerase chain reaction (PCR) cloning techniques can be used to construct the nucleic acid molecule.

For instance, the nucleic acid molecule can be cloned into a general purpose cloning vector such as pENTR (Gateway), pUC19, pBR322, pBluescript vectors (Stratagene Inc.) or pCR TOPO^{®} from Invitrogen Inc. The resultant nucleic acid construct (recombinant vector) carrying the nucleic acid molecule encoding the TCR can then be sub-cloned into expression vectors or viral vectors for protein expression, e.g. in mammalian cells. This may be for preparation of the TCR protein, or for expression in immune effector cells e.g. human T-cells or cell lines or other human immune effector cells. Further, the nucleic acid may be introduced into mRNA expression vectors for production of mRNA encoding the TCR. The mRNA may then be transferred into immune effector cells. Accordingly, another aspect of the invention provides a vector comprising a nucleic acid molecule of the invention.

An mRNA expression vector may alternatively be transcribed *in vitro* to produce mRNA encoding the TCR. For *in vitro* transcription (IVT) a template is first obtained. This may be a linearised mRNA expression vector. A vector may be linearised, for instance, using a restriction enzyme. Alternatively, the template may be obtained by PCR amplification of the expression cassette, or in any other way commonly known by the skilled person. The template is then purified and transcribed. Transcription may be performed using an IVT kit, such as a MEGAscript^{™} kit, a RiboMAX^{™} kit or a MAXIscript^{™} kit. DNA template may then be removed by DNase digestion of the sample, followed by purification of the mRNA. Methods of IVT are well-known to those skilled in the art.

A nucleic acid molecule of the invention may be introduced into a cell in a vector or as an isolated nucleic acid molecule or recombinant construct. Methods of heterologous gene expression are known in the art, both in terms of construct/vector preparation and in terms of introducing the nucleic acid molecule (vector or construct) into the cell. Thus, promoters and/or other expression control sequences suitable for use with mammalian cells, in particular T-cells, and appropriate vectors (e.g. viral vectors) are well known in the art.

Thus the nucleic acid molecule may be introduced or inserted into a vector. The term "vector" as used herein refers to a vehicle into which the nucleic acid molecule may be introduced (e.g. be covalently inserted) so as to bring about the expression of the TCR protein or mRNA and/or the cloning of the nucleic acid molecule. The vector may accordingly be a cloning vector or an expression vectors.

The nucleic acid molecule may be inserted into a vector using any suitable methods known in the art, for example, without limitation, the vector may be digested using appropriate restriction enzymes and then may be ligated with the nucleic acid molecule having matching restriction ends.

Expression vectors can contain a variety of control sequences, which refer to nucleic acid sequences necessary for the transcription and possibly translation of an operatively linked coding sequence in a particular host cell. In addition to control sequences that govern transcription and translation, vectors may contain additional nucleic acid sequences that serve other functions, including, for example, functions in replication or functions as selectable markers etc.

The expression vector should have the necessary 5' upstream and 3' downstream regulatory elements for efficient gene transcription and translation in its respective host cell, such as promoter sequences, examples of which include the CMV, PGK and EF1a promoters, the TATA box for ribosome recognition and binding, and a 3' UTR AATAAA (SEQ ID NO: 53) transcription termination sequence. Other suitable promoters include the constitutive 'early promoter' of simian virus 40 (SV40), the mouse mammary tumour virus (MMTV) promoter, the human immunodeficiency virus (HIV) long terminal repeat (LTR) promoter, the Moloney murine leukaemia virus (MoMuLV) promoter, the avian leukaemia virus (ALV) promoter, the Epstein-Barr virus (EBV) immediate-early promoter and the Rous sarcoma virus (RSV) promoter. Human gene promoters may also be used, including, but not limited to, the actin promoter, the myosin promoter, the haemoglobin promoter and the creatine kinase promoter. In certain embodiments inducible promoters are also contemplated as part of the vectors expressing the TCR. This provides a molecular switch capable of turning expression of the nucleic acid molecule on or off. Examples of inducible promoters include, but are not limited to, a metallothionein promoter, a glucocorticoid promoter, a progesterone promoter or a tetracycline promoter.

Further, the expression vector may contain 5' and 3' untranslated regulatory sequences that may function as enhancer sequences, and/or terminator sequences that can facilitate or enhance efficient transcription of the nucleic acid molecule.

Examples of vectors include plasmids, autonomously replicating sequences and transposable elements. Additional exemplary vectors include, without limitation, plasmids, phagemids, cosmids, artificial chromosomes such as a yeast artificial chromosome (YAC), a bacterial artificial chromosome (BAC) or a P1-derived artificial chromosome (PAC), bacteriophages such as lambda phage or M13 phage, and animal viruses. Examples of categories of animal viruses useful as vectors include, without limitation, retroviruses (including lentiviruses), adenoviruses, adeno-associated viruses, herpesviruses (e.g. herpes simplex virus), poxviruses, baculoviruses, papillomaviruses and papovaviruses (e.g. SV40). Examples of expression vectors are pCI-neo vectors (Promega) for expression in mammalian cells and pLenti4/V5-DEST^{™} and pLenti6/V5-DEST^{™} for lentivirus-mediated gene transfer and expression in mammalian cells.

In certain embodiments viral vectors are preferred. A viral vector can be derived from a retrovirus, particularly a lentivirus or a spumavirus/foamyvirus. As used herein, the term "viral vector" refers to a nucleic acid vector construct that includes at least one element of viral origin and has the capacity to be packaged into a viral vector particle. The viral vector can contain the nucleic acid molecule of the invention in place of nonessential viral genes. The vector and/or particle can be utilized for the purpose of transferring DNA, RNA or other nucleic acids into cells either *in vitro* or ex *vivo.*

Accordingly, a further aspect of the invention includes a viral particle comprising a nucleic acid molecule as defined and described herein, or a preparation or composition comprising such viral particles. Such a composition may also contain at least one physiologically acceptable carrier.

Numerous forms of viral vectors are known in the art. In certain embodiments, the viral vector is a retroviral vector or a lentiviral vector. The vector may be a self-inactivating vector in which the 3' LTR enhancer-promoter region, known as the U3 region, has been modified (e.g., by deletion or substitution) to prevent viral transcription beyond the first round of viral replication. Consequently, the vectors are capable of infecting and then integrating into the host genome only once, and cannot be passed further.

The retroviral vectors for use herein can be derived from any known retrovirus, e.g. Type C retroviruses, such as Moloney murine sarcoma virus (M-MSV), Harvey murine sarcoma virus (Ha-MuSV), mouse mammary tumour virus (MMTV), gibbon ape leukaemia virus (GaLV), feline leukaemia virus (FLV), spumaviruses, Friend virus, murine stem cell virus (MSCV) and Rous sarcoma virus (RSV); human T-cell leukaemia viruses such as HTLV-1 and HTLV-2; and the lentiviral family of retroviruses, such as the human immunodeficiency viruses HIV-1 and HIV-2, simian immunodeficiency virus (SIV), feline immunodeficiency virus (FIV), equine immunodeficiency virus (EIV), and other classes of retroviruses.

A lentiviral vector is derived from a lentivirus, a group (or genus) of retroviruses that give rise to slowly developing disease. Viruses included within this group include HIV (human immunodeficiency virus; including HIV type 1, and HIV type 2).

A retroviral packaging cell line (typically a mammalian cell line) may be used to produce viral particles, which may then be used for transduction of T-cells. Illustrative viral vectors are described in WO2002087341, WO2002083080, WO2002082908, WO2004000220 and WO2004054512. An exemplary retroviral vector is pMP71 as described in Wälchli *et al* 2011. Other suitable vectors include pBABE, pWZL, pMCs-CAG, pMXs-CMV, pMXs-EF1α, pMXs-IRES, pMXs-SRα and pMYs-IRES.

It is within the scope of the invention to include gene segments that cause immune effector cells carrying a vector or construct of the invention to be susceptible to negative selection *in vivo.* By "negative selection" is meant that the infused cell can be eliminated as a result of a change in the *in vivo* condition of the individual. The negatively selectable phenotype may result from the insertion of a gene that confers sensitivity to an administered agent, for example, a compound. Negatively selectable genes are known in the art, and include, *inter alia,* the following: the Herpes simplex virus type I thymidine kinase (HSV-I TK) gene (Wigler et al., Cell 11 (1):223-232, 1977) which confers ganciclovir sensitivity; the cellular hypoxanthinephosphribosyltransferase (HPRT) gene, the cellular adenine phosphoribosyltransferase (APRT) gene and bacterial cytosine deaminase, (Mullen et al., Proc. Natl. Acad. Sci. USA. 89:33-37 (1992)). A vector or construct of the invention may therefore comprise such a gene.

In some embodiments it may be useful to include in the vector or construct of the invention a positive marker that enables the selection of cells of the negatively selectable phenotype *in vitro,* e.g. selection of the genetically modified immune effector cells. The positively selectable marker may be a gene which, upon being introduced into the host cell, expresses a dominant phenotype permitting positive selection of cells carrying the gene. Genes of this type are known in the art, and include, *inter alia,* hygromycin-B phosphotransferase gene (hph) which confers resistance to hygromycin B, the amino glycoside phosphotransferase gene (neo or aph) from Tn5 which codes for resistance to the antibiotic G418, the dihydrofolate reductase (DHFR) gene, the adenosine deaminase gene (ADA) and the multi-drug resistance (MDR) gene.

Preferably, the positively selectable marker and the negatively selectable element are linked such that loss of the negatively selectable element necessarily also is accompanied by loss of the positively selectable marker. Even more preferably, the positively and negatively selectable markers are fused, so that loss of one obligatorily leads to loss of the other. An example of a fused polynucleotide that yields as an expression product a polypeptide that confers both the desired positive and negative selection features described above is a hygromycin phosphotransferase-thymidine kinase fusion gene (HyTK). Expression of this gene yields a polypeptide that confers hygromycin B resistance for positive selection *in vitro,* and ganciclovir sensitivity for negative selection *in vivo.* (See Lupton S. D., et al, Mol. and Cell. Biology 11:3374-3378, 1991.)

For cloning of the nucleic acid molecule the vector may be introduced into a host cell (e.g. an isolated host cell), and such "cloning host cells" containing a cloning vector of the invention form a further aspect of the invention. Suitable cloning host cells can include, without limitation, prokaryotic cells, fungal cells, yeast cells, or higher eukaryotic cells such as mammalian cells. Suitable prokaryotic cells for this purpose include, without limitation, eubacteria, such as Gram-negative or Gram-positive organisms, for example Enterobacteriaceae such as *Escherichia,* e.g. *E. coli, Enterobacter, Erwinia, Klebsiella, Proteus, Salmonella,* e.g. *Salmonella typhimurium, Serratia,* e.g. *Serratia marcescans,* and *Shigella,* as well as Bacilli such as *Bacillus subtilis* and *Bacilllus licheniformis, Pseudomonas* such as *P. aeruginosa,* and *Streptomyces.* A cloning host cell may alternatively contain an mRNA expression vector comprising the nucleic acid molecule.

The nucleic acid molecules or vectors are introduced into a host cell (e.g. a cloning host cell, production host cell or a T-cell) using transfection and/or transduction techniques known in the art. As used herein, the terms "transfection" and "transduction" refer to the processes by which an exogenous nucleic acid sequence is introduced into a host cell. The nucleic acid may be integrated into the host cell DNA or may be maintained extra-chromosomally. The nucleic acid may be maintained transiently or a may be stable. Transfection may be accomplished by a variety of means known in the art including but not limited to calcium phosphate-DNA co-precipitation, DEAE-dextran-mediated transfection, polybrene-mediated transfection, electroporation, microinjection, liposome fusion, lipofection, protoplast fusion, retroviral infection and biolistics. Transduction refers to the delivery of a gene(s) using a viral or retroviral vector by means of viral infection rather than by transfection. In certain embodiments, retroviral vectors are transduced by packaging the vectors into viral particles or virions prior to contact with a cell.

The invention also provides a host cell comprising a nucleic acid molecule or vector of the invention. Such a host cell may be any suitable host, including a cloning host, a production host or an immune effector cell. The host cell may be derived from any species, and indeed any domain of life, as appropriate for its function.

In one embodiment the invention provides an immune effector cell comprising a nucleic acid molecule or vector of the invention which encodes a full-length TCR of the invention. An "immune effector cell," is any cell of the immune system that has one or more effector functions (e.g., cytotoxic cell killing activity, secretion of cytokines, induction of ADCC and/or CDC). Representative immune effector cells thus include T lymphocytes, in particular cytotoxic T-cells (CTLs; CD8+ T-cells) and helper T-cells (HTLs; CD4+ T-cells). Other populations of T-cells are also useful herein, for example naive T-cells and memory T-cells. Other immune effector cells include NK cells, NKT cells, neutrophils, and macrophages. As noted above, immune effector cells also include progenitors of effector cells, wherein such progenitor cells can be induced to differentiate into an immune effector cells in vivo or in vitro. T-cells and NK cells represent preferred immune effector cells according to the invention.

The T-cell of the invention can be any T-cell. It may be a cytotoxic T-cell (a CD8⁺ T-cell), a helper T-cell (a CD4⁺ T-cell), a naive T-cell, a memory T-cell or any other type of T-cell. Preferably the T-cell is a CD8⁺ T-cell. As defined herein, a T-cell of the invention may also be an immature T-cell, such as a CD4⁻/CD8⁻ cell or a CD4⁺/CD8⁺ cells, or a progenitor of a T-cell.

The term "NK cell" refers to a large granular lymphocyte, being a cytotoxic lymphocyte derived from the common lymphoid progenitor which does not naturally comprise an antigen-specific receptor (e.g. a T-cell receptor or a B-cell receptor). NK cells may be differentiated by their CD3⁻, CD56⁺ phenotype. The term as used herein thus includes any known NK cell or any NK-like cell or any cell having the characteristics of an NK cell. Thus primary NK cells may be used or in an alternative embodiment, a NK cell known in the art that has previously been isolated and cultured may be used. Thus a NK cell-line may be used. A number of different NK cells are known and reported in the literature and any of these could be used, or a cell-line may be prepared from a primary NK cell, for example by viral transformation (Vogel et al. 2014, Leukemia 28:192-195). Suitable NK cells include (but are by no means limited to), in addition to NK-92, the NK-YS, NK-YT, MOTN-1, NKL, KHYG-1, HANK-1, or NKG cell lines. In a preferred embodiment, the cell is an NK-92 cell (Gong et al. 1994, Leukemia 8:652-658), or a variant thereof. A number of different variants of the original NK-92 cells have been prepared and are described or available, including NK-92 variants which are non-immunogenic. Any such variants can be used and are included in the term "NK-92". Variants of other cell lines may also be used.

An immune effector cell of the invention is preferably human. Such an immune effector cell may be derived from any human individual. Preferably, when the immune effector cell is for therapeutic use, it is an autologous immune effector cell: i.e. it is derived from the patient to be treated, which ensures histocompatibility and non-immunogenicity, meaning once genetically modified, it will not induce an immune response from the patient. Where the immune effector cell is a non-autologous cell for therapeutic use (i.e. it is a donor cell obtained from an individual other than the patient) it is preferred that it is non-immunogenic, such that it does not, when administered to a subject, generate an immune response which affects, interferes with, or prevents the use of the cells in therapy. An immune effector cell of the invention may thus be an ex *vivo* cell. It may alternatively or also be an *in vitro* cell.

Non-autologous immune effector cells may be naturally non-immunogenic if they are HLA-matched to the patient, i.e. they express the same HLA alleles. Non-autologous immune effector cells, including those which are not HLA-matched to the patient and would therefore be immunogenic, and those which are HLA-matched to the patient and may not be immunogenic, may be modified to eliminate expression of MHC molecules, or to only weakly express MHC molecules at their surface. Alternatively, such cells may be modified to express non-functional MHC molecules.

Any means by which the expression of a functional MHC molecule is disrupted is encompassed. Hence, this may include knocking out or knocking down a molecule of the MHC complex, and/or it may include a modification which prevents appropriate transport to and/or correct expression of an MHC molecule, or of the whole complex, at the cell surface.

In particular, the expression of one or more functional MHC class-I proteins at the surface of an immune effector cell of the invention may be disrupted. In one embodiment the immune effector cells may be human cells which are HLA-negative, such as cells in which the expression of one or more HLA molecules is disrupted (e.g. knocked out), e.g. molecules of the HLA Class I MHC complex.

In a preferred embodiment, disruption of Class-I MHC expression may be performed by knocking out the gene encoding β₂-microglobulin (β₂-m), a component of the mature Class-I MHC complex. Expression of β₂-m may be eliminated through targeted disruption of the β₂-m gene, for instance by site-directed mutagenesis of the β₂-m promoter (to inactivate the promoter), or within the gene encoding the β₂-m protein to introduce an inactivating mutation that prevents expression of the β₂-m protein, e.g. a frame-shift mutation or premature 'STOP' codon within the gene. Alternatively, site-directed mutagenesis may be used to generate non-functional β₂-m protein that is not capable of forming an active MHC protein at the cell surface. In this manner the β₂-m protein or MHC may be retained intracellularly, or may be present but non-functional at the cell surface.

Immune effector cells may alternatively be irradiated prior to being administered to a subject. Without wishing to be bound by theory, it is thought that the irradiation of cells results in the cells only being transiently present in a subject, thus reducing the time available for a subject's immune system to mount an immunological response against the cells. Whilst such cells may express a functional MHC molecule at their cell surface, they may also be considered to be non-immunogenic. Radiation may be from any source of α, β or γ radiation, or may be X-ray radiation or ultraviolet light. A radiation dose of 5-10 Gy may be sufficient to abrogate proliferation, however other suitable radiation doses may be 1-10, 2-10, 3-10, 4-10, 6-10, 7-10, 8-10 or 9-10 Gy, or higher doses such as 11, 12, 13, 14, 15 or 20 Gy. Alternatively, the cells may be modified to express a 'suicide gene', which allows the cells to be inducibly killed or prevented from replicating in response to an external stimulus.

Thus, an immune effector cell according to the invention may be modified to be non-immunogenic by reducing its ability, or capacity, to proliferate, that is by reducing its proliferative capacity.

The modified immune effector cells of the invention may also be subject to modification in other ways, for example to alter or modify other aspects of cell function or behaviour, and/or to express other proteins. For instance, the cells may be modified to express a homing receptor, or localisation receptor, which acts to target or improve the localisation of the cells to a particular tissue or location in the body.

The present invention also provides methods for making the immune effector cells which express the TCR as described herein. In one embodiment, the method comprises transfecting or transducing T-cells isolated from a subject (who may be the patient or a donor) such that the T-cells express one or more TCR as described herein. In certain embodiments, the T-cells are isolated from a subject and modified by introduction of the nucleic acid molecule without further manipulation *in vitro.* Such cells can then be directly re-administered into the subject. In further embodiments, the T-cells are first activated and stimulated to proliferate *in vitro* (such activation and stimulation to proliferate may be referred to as expansion) prior to being modified to express a TCR. In this regard, the T-cells may be cultured before or after being genetically modified (i.e. transduced or transfected to express a TCR as described herein).

T-cells can be obtained from a number of sources, including peripheral blood mononuclear cells (PBMCs), bone marrow, lymph node tissue, cord blood, thymus tissue, tissue from a site of infection, ascites, pleural effusion, spleen tissue and tumours. In certain embodiments, T-cells can be obtained from a unit of blood collected from the subject using any number of techniques known to the skilled person, such as FICOLL^{™} separation. In one embodiment, cells from the circulating blood of a subject are obtained by apheresis. The apheresis product typically contains lymphocytes, including T-cells, monocytes, granulocytes, B-cells, other nucleated white blood cells, red blood cells, and platelets. In one embodiment, the cells collected by apheresis may be washed to remove the plasma fraction and to place the cells in an appropriate buffer or media for subsequent processing. In one embodiment of the invention, the cells are washed with PBS. In an alternative embodiment, the washed solution lacks calcium and/or magnesium or may lack many if not all divalent cations. As would be appreciated by those of ordinary skill in the art, a washing step may be accomplished by methods known to those in the art, such as by using a semiautomated flowthrough centrifuge. For example, the Cobe 2991 cell processor, the Baxter CytoMate, or the like. After washing, the cells may be resuspended in a variety of biocompatible buffers or other saline solution with or without buffer. In certain embodiments, the undesirable components of the apheresis sample may be removed in the cell directly resuspended culture media.

In certain embodiments, T-cells are isolated from PBMCs. PBMCs may be isolated from buffy coats obtained by density gradient centrifugation of whole blood, for instance centrifugation through a LYMPHOPREP^{™} gradient, a PERCOLL^{™} gradient or a FICOLL^{™} gradient. T-cells may be isolated from PBMCs by depletion of the monocytes, for instance by using CD14 DYNABEADS^{®}. In some embodiments, red blood cells may be lysed prior to the density gradient centrifugation.

A specific subpopulation of T-cells, such as CD28⁺, CD4⁺, CD8⁺, CD45RA⁺ or CD45RO⁺ T-cells, can, if desired, be further isolated by positive or negative selection techniques. For example, enrichment of a T-cell population by negative selection can be accomplished with a combination of antibodies directed to surface markers unique to the negatively selected cells. One method for use herein is cell sorting and/or selection via negative magnetic immunoadherence or flow cytometry that uses a cocktail of monoclonal antibodies directed to cell surface markers present on the cells negatively selected. For example, to enrich for CD4⁺ cells by negative selection, a monoclonal antibody cocktail typically includes antibodies to CD14, CD20, CDIIb, CD16, HLA-DR and CD8. Flow cytometry and cell sorting may also be used to isolate cell populations of interest for use in the present invention.

In certain embodiments, both cytotoxic and helper T-cells can be sorted into naïve, memory, and effector T-cell subpopulations either before or after genetic modification and/or expansion. CD8⁺ T-cells can be obtained by using standard methods as described above. In some embodiments, CD8⁺ T-cells are further sorted into naive, central memory, and effector cells by identifying cell-surface antigens that are associated with each of those types of CD8⁺ T-cells. Memory T-cells may be present in both CD62L⁺ and CD62L⁻ subsets of CD8⁺ peripheral blood T-cells. T-cells are sorted into CD62L⁻/CD8⁺ and CD62L⁺/CD8⁺ fractions after staining with anti-CD8 and anti-CD62L antibodies. Phenotypic markers of central memory T-cells (TCM) may include expression of CD45RO, CD62L, CCR7, CD28, CD3 and CD127, and lack of expression of granzyme B. TCMs may be CD45RO⁺/CD62L⁺/CD8⁺ T-cells. Effector T-cells may be negative for CD62L, CCR7, CD28, and CD127 expression, and positive for granzyme B and perforin expression. Naive CD8⁺ T-cells may be characterised by the expression of phenotypic markers of naive T-cells including CD62L, CCR7, CD28, CD3, CD127 and CD45RA.

Isolated immune effector cells can be modified following isolation, or they can be activated and expanded (or, in the case of progenitors, differentiated) *in vitro* prior to being modified. In an embodiment, the cells are modified by introduction of the nucleic acid molecules of the invention and then are activated and expanded *in vitro.* In another embodiment, the cells are activated and expanded *in vitro* then modified by introduction of the nucleic acid molecules of the invention. Methods for activating and expanding T-cells are known in the art and are described, for example, in US6905874, US6867041, US6797514, and WO2012079000. Generally, such methods include contacting PBMC or isolated T-cells with a stimulatory agent and co-stimulatory agent, such as anti-CD3 and anti-CD28 antibodies, generally attached to a bead or other surface (for instance in the form of CD3/CD28 DYNABEADS^{®}), in a culture medium supplemented with appropriate cytokines, such as IL-2. A bead with both anti-CD3 and anti-CD28 antibodies attached serves as a surrogate antigen presenting cell (APC). In other embodiments, the T-cells may be activated and stimulated to proliferate with feeder cells and appropriate antibodies and cytokines using methods such as those described in US6040177, US5827642 and WO2012129514.

In one embodiment, T-cells are transduced or transfected with a nucleic acid molecule in accordance with the invention. Methods of transduction and transfection are described above. The nucleic acid molecule of the invention may be a vector comprising the nucleic acid molecule of the invention (i.e. one which encodes a TCR molecule of the invention). Alternatively, it may be an mRNA molecule encoding a TCR molecule of the invention.

In another embodiment, CD34⁺ cells are transduced or transfected with a nucleic acid molecule in accordance with the invention. In certain embodiments, the modified (e.g. transfected or transduced) CD34⁺ cells differentiate into mature immune effector cells in vivo following administration into a subject, generally the subject from whom the cells were originally isolated. In another embodiment, CD34⁺ cells may be stimulated in vitro prior to or after introduction of the nucleic acid molecule, with one or more of the following cytokines: Flt-3 ligand (FL), stem cell factor (SF), megakaryocyte growth and differentiation factor (TPO), IL-3 and IL-6 according to the methods known in the art.

In another embodiment, the invention provides a production host cell which comprises a nucleic acid molecule or vector of the invention which encodes a soluble TCR of the invention. The production host cells are suitable for expression and production of the soluble TCR. The production host cell may be any cell suitable for protein production. For instance, the production host cell may be a prokaryotic cell, in particular a bacterial cell, such as a Gram-negative bacterial cell (e.g. *Escherichia coli*) or a Gram-positive bacterial cell (e.g. *Bacillus subtilis*)*.* The production host cell is preferably however a eukaryotic cell. A eukaryotic production host cell of the invention may be a simple eukaryotic cell, such as a yeast or fungal cell. Preferably, the eukaryotic production host cell is an animal cell. The animal cell may be an insect cell or any other animal cell, but is preferably a mammalian cell. For instance, the mammalian production host cell may be a primate cell, particularly a human cell, or a rodent cell: for instance it may be a HEK-293, HEK-293T, COS (e.g. COS-7) or CHO cell. Mammalian production host cells, particularly human production host cells are preferred, as appropriate post-translational modifications are made when the soluble TCR is expressed in a human cell. A person skilled in the art can easily select an appropriate production host cell.

The invention also provides a TCR molecule as defined herein, in particular a soluble TCR molecule as defined herein. The soluble TCR of the invention is a protein or protein complex which comprises a truncated α-chain domain and a truncated β-chain domain, as defined above. Soluble TCRs are described in detail in Walseng et al. (2015), PLoS ONE 10(4): e0119559.

The soluble TCR of the invention is preferably encoded as a single chain. Single chain soluble TCRs are described above and, as detailed, such scTCRs preferably include self-cleaving linker sequences between the α- and β-chain domains, and thus yield separate α- and β-chains.

Thus, the soluble TCR of the invention is preferably a TCR complex comprising an α-chain and a β-chain, which constitute separate polypeptide chains. The soluble TCR of the invention is a TCR encoded by a nucleic acid molecule of the invention, as defined above. As discussed above, the variable regions of the α- and β-chains are encoded and synthesised with leader sequences which direct the chains for insertion into the membrane, or in the case of a soluble TCR, secretion. These leader sequences are cleaved upon secretion. A TCR chain which comprises a leader sequence is known as immature, while one from which its leader has been cleaved is known as mature. The soluble TCR of the invention is preferably a mature soluble TCR, in which the leader sequences of the α- and β-chains are not present.

The soluble TCR of the invention is encoded by a nucleic acid molecule as defined above. Thus in a preferred embodiment, the soluble TCR α-chain is encoded with a variable region with the sequence set forth in SEQ ID NO: 8. As described above, the leader sequence of the Radium-1 α-chain is set forth in SEQ ID NO: 50, which corresponds to amino acids 1-20 of SEQ ID NO: 8. The mature form of the variable region of the Radium-1 α-chain (i.e. the variable region without the leader sequence) has the sequence set forth in SEQ ID NO: 72. The soluble TCR of the invention comprises an α-chain comprising a variable region comprising or consisting of the amino acid sequence set forth in SEQ ID NO: 72.

The truncated constant region of the soluble TCR α-chain has the sequence of SEQ ID NO: 60 or SEQ ID NO: 61, as described above. The α-chain of the soluble TCR of the invention thus has a variable region with the sequence of SEQ ID NO: 72 and a constant region with the sequence of SEQ ID NO: 60 or SEQ ID NO: 61. These α-chain sequences are set forth in SEQ ID NOs: 73 and 74, respectively. The soluble TCR of the invention thus preferably comprises an α-chain comprising or consisting of the amino acid sequence set forth in SEQ ID NO: 73 or SEQ ID NO: 74.

In another preferred embodiment, the soluble TCR β-chain is encoded with a variable region with the sequence set forth in SEQ ID NO: 13. As also described above, the leader sequence of the Radium-1 β-chain is set forth in SEQ ID NO: 51, which corresponds to amino acids 1-16 of SEQ ID NO: 13. The mature form of the variable region of the Radium-1 β-chain (i.e. the variable region without the leader sequence) has the sequence set forth in SEQ ID NO: 75. The soluble TCR of the invention comprises a β-chain comprising a variable region comprising or consisting of the amino acid sequence set forth in SEQ ID NO: 75.

The truncated constant region of the soluble TCR β-chain has the sequence of SEQ ID NO: 62 or SEQ ID NO: 63, as described above. The β-chain of the soluble TCR of the invention thus has a variable region with the sequence of SEQ ID NO: 75 and a constant region with the sequence of SEQ ID NO: 62 or SEQ ID NO: 63. These β-chain sequences are set forth in SEQ ID NOs: 76 and 77, respectively. The soluble TCR of the invention thus preferably comprises a β-chain comprising or consisting of the amino acid sequence set forth in SEQ ID NO: 76 or SEQ ID NO: 77.

In another preferred embodiment, as discussed above, the α- and β-chains of the soluble TCR each comprise a leucine zipper sequence at the C-terminus.

In another preferred embodiment, at least one chain of the soluble TCR is encoded with a purification tag. Such a tag may be any suitable tag known to the skilled person, e.g. a FLAG-tag, a His-tag, an HA-tag, a Strep-tag, an S-tag, or a Myc-tag, glutathione S-transferase (GST), maltose-binding protein (MBP), etc. The tag is preferably located at the C-terminus of either the α- or β-chain, most preferably the β-chain. Thus, a soluble TCR of the invention may comprise a purification tag in its α- and/or β-chain, preferably at the C-terminus of the chain(s). The TCR chain may be encoded with a linker and/or a protease cleavage site between the main chain sequence (i.e. the variable domain and the segment of the constant domain which is present, and where present the leucine zipper domain) and the purification tag. Appropriate protease cleavage sites are well-known to the skilled person and include thrombin, factor Xa, enterokinase, human rhinovirus (HRV) 3C and tobacco etch virus (TEV) cleavage sites. In a particular embodiment, the β-chain of the soluble TCR of the invention comprises a His-tag joined to the chain via Gly-Gly-Gly linker.

The sequence of the α-chain of SEQ ID NO: 74 with a His-tag joined to its C-terminus via a Gly-Gly-Gly linker is shown in SEQ ID NO: 78; the sequence of the β-chain of SEQ ID NO: 77 with a His-tag joined to its C-terminus via a Gly-Gly-Gly linker is shown in SEQ ID NO: 79. In preferred embodiments of the invention, the soluble TCR α-chain comprises or consists of the amino acid sequence of SEQ ID NO: 78, and/or the soluble TCR β-chain comprises or consists of the amino acid sequence of SEQ ID NO: 79.

As detailed above, in a preferred embodiment of the invention, the soluble TCR is encoded as an scTCR with the α- and β-chain domains separated by a 2A linker. 2A linkers are discussed above, and as detailed above these sequences undergo co-translational cleavage between their final proline residue and penultimate glycine residue. The terminal proline of 2A linker thus forms the N-terminal residue of the downstream polypeptide, while all the other residues of the 2A linker form the C-terminus of the upstream polypeptide. In the preferred scTCRs of the invention, the α-chain domain forms the upstream polypeptide and the β-chain domains forms the downstream polypeptide. As detailed above, the N-terminus of each chain of a TCR is a leader sequence which is cleaved during maturation of the polypeptide. In the scTCRs of the invention, the terminal proline residue of the 2A linker will form the N-terminal residue of the β-chain, and will be cleaved from the chain with the leader sequence. The residue will not, therefore be present in the mature β-chain. However, the other residues of the 2A linker will form the C-terminus of the α-chain and will be present in the mature α-chain.

Thus in a particular embodiment of the invention, the soluble TCR comprises an α-chain in which the C-terminus is formed from all but the final residue of the 2A peptide. In particular, the C-terminus of the α-chain (i.e. the final 25 residues) may be amino acids 1-25 of the 2A sequence presented in SEQ ID NO: 18. In this embodiment, the soluble TCR α-chain may in particular comprise or consist of the amino acid sequence set forth in SEQ ID NO: 81. The amino acid sequence set forth in SEQ ID NO: 81 is that of SEQ ID NO: 73 (the mature truncated Radium-1 α-chain) with a C-terminal addition of residues 1-25 of SEQ ID NO: 18. In another embodiment, the soluble TCR α-chain comprises or consists of the amino acid sequence set forth in SEQ ID NO: 82. The amino acid sequence set forth in SEQ ID NO: 82 is that of SEQ ID NO: 74 (the mature truncated cysteine-modified Radium-1 α-chain) with a C-terminal addition of residues 1-25 of SEQ ID NO: 18.

As described above, the α- and β-chains of the soluble TCR are joined to each other: this is a requirement as the complex will otherwise separate in solution. As discussed above, the α- and β-chains may be covalently joined, e.g. via a disulphide bond, or non-covalently joined by leucine zipper domains located at the C-termini of the chains.

As detailed above, all α- and β- TCR chains are expressed with an N-terminal leader sequence which directs the polypeptide chain to the membrane for insertion/secretion. The α-chain of the soluble TCR of the invention is encoded with such a sequence, and in the soluble TCR of the invention may comprise an N-terminal leader sequence or may not comprise such a sequence. Equivalently, the β-chain of the soluble TCR of the invention is encoded with such a sequence, and in the soluble TCR of the invention may comprise an N-terminal leader sequence or may not comprise such a sequence. Such α- and β-chains, and their leader sequences are described above.

The soluble TCR of the invention may be produced by any method known in the art, in particular by a host production cell as defined above. The soluble TCR may be expressed using standard protein expression techniques under standard conditions. The leader sequences of the α- and β-chains target the chains for export from the cell in which they are expressed (e.g. the production host cell). The α- and β-chains are thus exported from the production host cell into the extra-cellular milieu, where the chains form a complex, e.g. via a disulphide bond or dimerisation of leucine zipper domains. The soluble TCR complex may then be purified: firstly, the cell culture can be centrifuged and the supernatant isolated. The soluble TCR complex can then be purified from the supernatant by affinity chromatography, using the purification tag at the C-terminus of the α- and/or β-chain. If a protease cleavage site is present N-terminal to the purification tag, the tag may be cleaved from the chain using the appropriate protease.

If desired, the soluble TCR may be multimerised to form a soluble TCR multimer. Such multimers form an aspect of the invention. For instance, multimerisation may be performed by conjugation of TCR molecules to nanobeads, e.g. magnetic nanobeads. Methods for such conjugations are well-known in the art. In another embodiment, soluble TCR complexes can be biotinylated and conjugated to streptavidin, to yield tetrameric soluble TCR complexes. In order to biotinylate a soluble TCR complex, one of the TCR chains should be expressed with a BirA sequence (SEQ ID NO: 59) at its C-terminus. Biotinylation of the TCR complex at the BirA sequence can then be performed using *E. coli* BirA (biotin ligase). Once biotinylated, the soluble TCR complexes can be incubated with streptavidin to produce soluble TCR tetramers.

In a particularly preferred embodiment of the invention, the soluble TCR is conjugated to a toxin. The chosen toxin is a toxin which, alone, is unable to enter, kill or otherwise disrupt a human cell but, when taken up by a human cell via a conjugated molecule, is able to exert its toxic effects. Such a toxin will thus only be taken up by, and exert its target effects on, a cell bound by the soluble TCR of the invention, into which the soluble TCR is taken up. The toxin may be any known appropriate cytotoxic species, i.e. it may be any suitable cytotoxin. By "cytotoxin" as used herein is meant any toxin which inhibits the growth and/or viability of a cell. Growth includes the division of a target cell (i.e. a cell into which it enters). The toxin may thus be any toxin which reduces or has a negative impact on the viability or survival of a cell and in particular includes any toxin which induces death of a target cell, e.g. the toxin may induce apoptosis or necrosis of a target cell.

Such a toxin may be a peptide toxin lacking a targeting domain. For instance, it may be a peptide toxin which natively lacks a targeting domain, or it may be a peptide toxin modified relative to its native form to remove its targeting domain. Examples of such toxins include saporin and gelonin, which are ribosome-inactivating proteins (RIPs) of the same family as e.g. ricin, but which are unable to cross the plasma membrane of a cell. Similarly, the enzymatic domains (i.e. catalytic domains) of a cytotoxin of a pathogen may be used, such as the enzymatic domain of a bacterial cytotoxin, e.g. the enzymatic domain of diphtheria toxin, *Pseudomonas* exotoxin A or a Clostridial cytotoxin, e.g. TcsL of *Clostridium sordellii.*

The soluble TCR of the invention may be encoded as a fusion protein, with a toxin located at the C-terminus of either the α- or β-chain. Alternatively, the toxin may be conjugated to the soluble TCR using any suitable method known in the art. For instance, the soluble TCR molecule may be biotinylated on either its α- or β-chain and conjugated to streptavidin-conjugated toxin (or *vice versa*)*.* Other suitable methods are known to those skilled in the art.

The invention provides a modified immune effector cell for use in the treatment of cancer, wherein the cancer expresses a mutated TGFβRII protein which comprises SEQ ID NO: 1, the modified immune effector cell expressing a TCR as disclosed herein. For example, the modified immune effector cells may be prepared from PBMCs obtained from a patient diagnosed with MSI+ colorectal cancer. Standard procedures may be used for storage, e.g. cryopreservation, of the modified immune effector cells and/or preparation for use in a human or other subject.

The modified immune effector cells expressing the TCR of the invention can be utilized in methods and compositions for adoptive cell transfer immunotherapy in accordance with known techniques. In some embodiments, the cells are formulated by first harvesting them from their culture medium, and then washing and concentrating the cells in a medium and container system suitable for administration (a "pharmaceutically acceptable" carrier) in a treatment-effective amount. Suitable infusion medium can be any isotonic medium formulation, typically normal saline, Normosol R (Abbott) or Plasma-Lyte A (Baxter), but also 5% dextrose in water or Ringer's lactate can be utilized. The infusion medium can be supplemented with human serum albumin.

A treatment-effective amount of cells in the composition is at least 2 cells (for example, at least 1 CD8⁺ central memory T-cell and at least 1 CD4⁺ helper T-cell subset) or is more typically greater than 10² cells, and up to 10⁶, up to and including 10⁸ or 10⁹ cells and can be more than 10¹⁰ cells. The number of cells will depend upon the ultimate use for which the composition is intended as will the type of cells included therein. For uses provided herein, the cells are generally in a volume of a litre or less, 500 ml or less, even 250 ml or 100 ml or less. Hence the density of the desired cells is typically greater than 10⁶ cells/ml and generally is greater than 10⁷ cells/ml, generally 10⁸ cells/ml or greater. The clinically relevant number of immune cells can be apportioned into multiple infusions that cumulatively equal or exceed 10⁵, 10⁶, 10⁷, 10⁸, 10⁹, 10¹⁰, 10¹¹, or 10¹² cells. For example, 2, 3, 4, 5, 6 or more separate infusions may be administered to a patient, at intervals of 24 or 48 hours, or every 3, 4, 5, 6 or 7 days. Infusions may also be spaced at weekly, fortnightly or monthly intervals, or intervals of 6 weeks or 2, 3, 4, 5, or 6 months. It is also possible that yearly infusions may be administered. In some aspects of the present invention, since all the infused cells are redirected to a particular target antigen (namely the TGFβRII frameshift peptide with the sequence of SEQ ID NO: 1), lower numbers of cells, in the range of 10⁶/kilogram (10⁶-10⁸ per patient) may be administered. The cell compositions may be administered multiple times at dosages within these ranges. If desired, the treatment may also include administration of mitogens (e.g., PHA) or lymphokines, cytokines, and/or chemokines (e.g., IFN-γ, IL-2, IL-12, TNF-alpha, IL-18, and TNF-beta, GM-CSF, IL-4, IL-13, Flt3-L, RANTES, MΓPTα, etc.) to enhance induction of the immune response.

The immune effector cells of the present invention, which express a TCR molecule of the invention, may be administered either alone, or as a pharmaceutical composition in combination with diluents and/or with other components such as IL-2 or other cytokines or cell populations. Briefly, pharmaceutical compositions of the present invention comprise a TCR-expressing immune effector cell, e.g. T-cell, population, as described herein, in combination with one or more pharmaceutically or physiologically acceptable carriers, diluents or excipients. Such compositions may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminium hydroxide); and preservatives. Compositions of the present invention are preferably formulated for intravenous administration.

As noted elsewhere with regard to *in vivo* selectable markers for use in the vectors encoding the TCR, adverse events may be minimized by transducing the immune effector cells expressing the TCR with a suicide gene, such as inducible caspase 9 or a thymidine kinase, before, after or at the same time as the cells are modified with the nucleic acid molecule of the present invention. Alternatively, as noted with regard to the TCR of the invention, the TCR may comprise a tag, particularly a double Myc-tag, allowing targeted killing of the T-cells of the invention using an antibody which recognises the tag used (such as an anti-Myc antibody).

The present invention also provides a soluble TCR as defined herein, and a composition comprising such a soluble TCR, for use in therapy, in particular for use in the treatment of cancer, wherein the cancer expresses a mutated TGFβRII protein which comprises SEQ ID NO: 1.

Liquid pharmaceutical compositions, whether they be solutions, suspensions or other like form, may include one or more of the following: sterile diluents such as water, saline solution (preferably physiological saline), Ringer's solution, isotonic sodium chloride, fixed oils such as synthetic mono- or diglycerides (which may serve as the solvent or suspending medium), polyethylene glycols, glycerin, propylene glycol or other solvents; antibacterial agents such as benzyl alcohol or methyl paraben; antioxidants such as ascorbic acid or sodium bisulfite; chelating agents such as ethylenediaminetetraacetic acid (EDTA); buffers such as acetates, citrates or phosphates, and agents for the adjustment of tonicity such as sodium chloride or dextrose. The parenteral preparation can be enclosed in ampoules, disposable syringes or multiple dose vials made of glass or plastic. An injectable pharmaceutical composition is preferably sterile.

Pharmaceutical compositions of the present invention may be administered in a manner appropriate to the disease to be treated (or prevented). The quantity and frequency of administration will be determined by such factors as the condition of the patient, and the type and severity of the patient's disease, although appropriate dosages may be determined by clinical trials.

The immune response induced in a subject by administering immune effector cells expressing the TCR of the invention, as described herein, may include cellular immune responses mediated by cytotoxic T-cells or NK cells capable of killing target cells (i.e. tumour cells expressing the TGFβRII frameshift peptide of SEQ ID NO: 1), regulatory T-cells and helper T-cells. Humoral immune responses, mediated primarily by helper T-cells capable of activating B-cells and thus generating an antibody response, may also be induced.

Administration to a subject of a soluble TCR carrying a toxin, as described herein, leads to uptake of the TCR/toxin conjugates by target cells, resulting in direct and selective killing of the target cells by the toxin.

When an "effective amount" is indicated, the precise amount of the compositions to be administered can be determined by a physician with consideration of individual differences in age, weight, extent of malignancy, and general condition of the patient (subject). The optimal dosage and treatment regime for a particular patient can readily be determined by one skilled in the art of medicine by monitoring the subject for signs of disease and adjusting the treatment accordingly.

Thus, the present invention provides for the treatment of a subject diagnosed with, or suspected of having, or at risk of developing, a cancer which expresses a frameshift mutant of TGFβRII, said frameshift mutant comprising within its sequence the neopeptide of SEQ ID NO: 1. Such a cancer is likely to be an MSI+ cancer, though may be non-MSI+. The cancer may be any cancer which expresses the neopeptide of SEQ ID NO: 1. In particular, non-limiting embodiments the cancer is colorectal cancer, gastric cancer, liver cancer, ampullary carcinoma, endometrial cancer, pancreatic cancer or leukaemia. The cancer may particularly be in a patient suffering from Lynch Syndrome/HNPCC. In a particular embodiment, the invention provides a treatment for colorectal cancer in a subject with HNPCC.

The immune effector cells and/or soluble TCRs of the invention may be administered in combination with one or more other therapeutic agents, which may include any other known cancer treatments, such as radiation therapy, chemotherapy, transplantation, immunotherapy, hormone therapy, photodynamic therapy, etc. The immune effector cells and soluble TCRs of the invention may be administered in combination together. The compositions may also be administered in combination with antibiotics or other therapeutic agents, including e.g. cytokines (e.g. IL-1, IL-2, IL-3, IL-4, IL-5, IL-6, IL-7, IL-8, IL-9, IL-10, IL-11, IL-12, IL-13, IL-15 and IL-17), growth factors, steroids, NSAIDs, DMARDs, antiinflammatories, analgesics, chemotherapeutics (e.g. monomethyl auristatin E, fludarabine, gemcitabine, capecitabine, methotrexate, taxol, taxotere, mercaptopurine, thioguanine, hydroxyurea, cytarabine, cyclophosphamide, ifosfamide, nitrosoureas, cisplatin, carboplatin, oxaliplatin, mitomycin, dacarbazine, procarbazine, etoposide, teniposide, campathecins, bleomycin, doxorubicin, idarubicin, daunorubicin, dactinomycin, plicamycin, mitoxantrone, L-asparaginase and 5-fluorouracil), radiotherapeutics, immune checkpoint inhibitors (e.g. Tremelimumab, Ipilimumab, Nivolumab, MK-3475, Urelumab, Bavituximab, MPDL3280A and MEDI4736), small molecule inhibitors or other active and ancillary agents.

Immune effector cells, soluble TCRs and compositions of the present invention are therefore for use in therapy, particularly cancer therapy for treating a cancer expressing a mutated TGFβRII protein which comprises SEQ ID NO: 1, more particularly therapy for treating such an MSI+ cancer or colorectal cancer. Immune effector cells of the present invention (and compositions comprising them) may in particular be used in adoptive cell transfer therapy (also known as adoptive cell therapy), as described herein.

The term 'target cell' refers to any cell which is to be killed or abrogated by the modified immune effector cells or soluble TCRs of the invention. As noted above, it will be a cancer (or alternatively expressed, tumour) cell which expresses a frameshift mutant of TGFβRII, said frameshift mutant comprising within its sequence the neopeptide of SEQ ID NO: 1. A target cell may in certain embodiments be a MSI+ cancer cell, or a colorectal or gastric cancer cell, or indeed a cancer cell from any of the cancers listed above.

Cancer is defined broadly herein to include any neoplastic condition, whether malignant, pre-malignant or non-malignant. Generally, however, it may be a malignant condition. Both solid and non-solid tumours are included and the term "cancer cell" may be taken as synonymous with "tumour cell".

In one embodiment of the present invention the cells, soluble TCR and/or compositions of the invention may be administered to a subject intravenously. In an alternative embodiment the cells, soluble TCR and/or composition may be administered directly into a tumour via intratumoural injection.

The subject to be treated using the cells of the present invention may be any species of mammal. For instance, the subject may be any species of domestic pet, such as a mouse, rat, gerbil, rabbit, guinea pig, hamster, cat or dog, or livestock, such as a goat, sheep, pig, cow or horse. In a further preferred embodiment of the invention the subject may be a primate, such as a monkey, gibbon, gorilla, orang-utang, chimpanzee or bonobo. However, in a preferred embodiment of the invention the subject is a human. It is contemplated that immune effector cells for use in the present invention may be obtained from any species of mammal, however, in a preferred embodiment the immune effector cells will be from the same species of mammal as the subject to be treated.

The present invention may be more fully understood from the non-limiting Examples below and in reference to the drawings, in which:

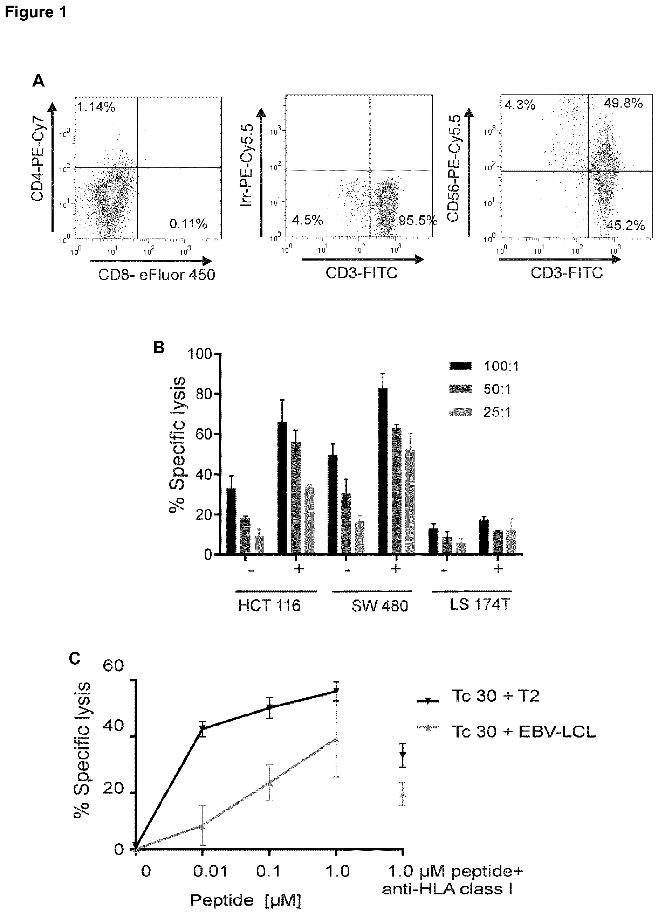

**Figure** 1 shows that the originally-isolated T-cell clones are TGFβRII frameshift mutation-specific, CD8⁻/CD4⁻/CD56⁺ and kill target cells in a dose-dependent manner.
   (a) shows that the original T-cell clone is CD8⁻CD4⁻and CD56⁺.
   (b) shows the results of ⁵¹Cr-release assays demonstrating specific lysis by T-cell clone 26 of colon cancer cell lines, loaded with 1 µM p573 peptide (which has the sequence of SEQ ID NO: 1) or control peptide I540 (which has the sequence of SEQ ID NO: 54), at various effector-to-target (E:T) ratios as indicated.
   (c) shows the results of ⁵¹Cr-release assays demonstrating specific lysis by the T-cell clone of autologous EBV-LCL (Epstein Barr Virus-transformed lymphoblastoid cell line) or T2 cells, loaded with titrated concentrations of p573 peptide. Blocking with anti-HLA class I at the highest peptide concentration is also shown. The E:T ratio was 25:1. The results shown are representative of three independent experiments.

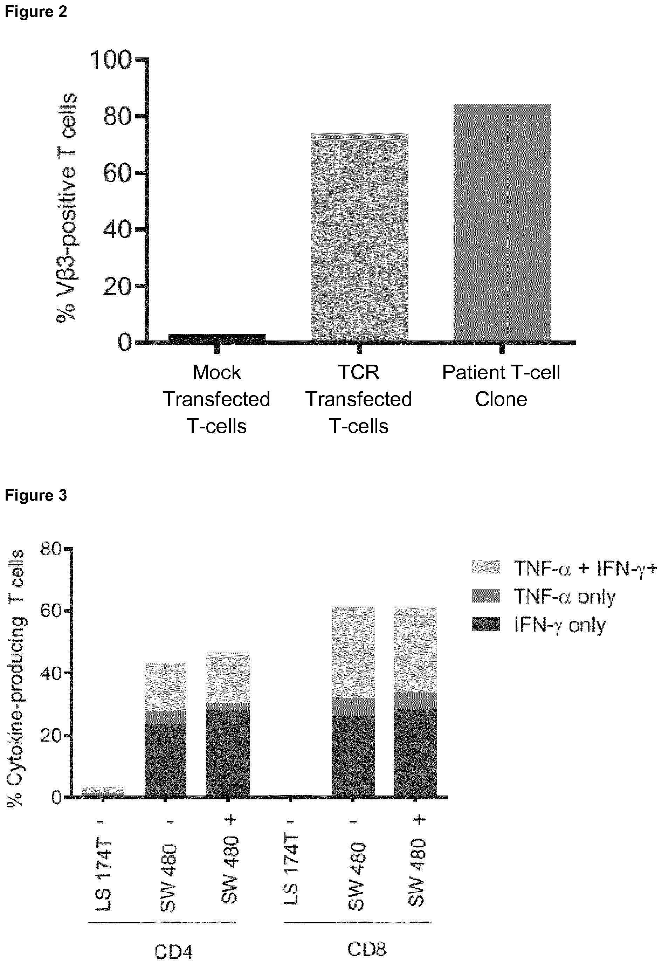

**Figure 2** shows TGFβRII -1A frameshift mutation (TGFβRII^{mut})-specific TCR expression in transfected *in* vitro-expanded T-cells. TGFβRII^{mut}-specific TCR expression corresponds T-cells which are Vβ3⁺ (Vβ3 = TCR β-chain variable domain TRBV28). Mock transfected T-cells, TCR mRNA transfected T-cells and the original T-cell clone were all tested.
**Figure 3** shows that both TCR-transfected CD4⁺ and TCR-transfected CD8⁺ T-cells produce IFN-γ and TNF-α in response to colon cancer cell lines harbouring the TGFβRII -1A frameshift mutation. T-cells were transfected with TCR mRNA and left overnight before co-incubation with colon cancer cell lines LS174T and SW480 expressing mutated TGFβRII. LS174T is HLA Class I negative whereas SW480 expresses HLA-A2. SW480 cells were either loaded (+) or not loaded (-) with the TGFβRII frameshift peptide p573. After overnight stimulation, cells were stained intracellularly for cytokine production. Plots show CD4 or CD8 gated T-cells as indicated. The results shown are representative of three independent experiments.

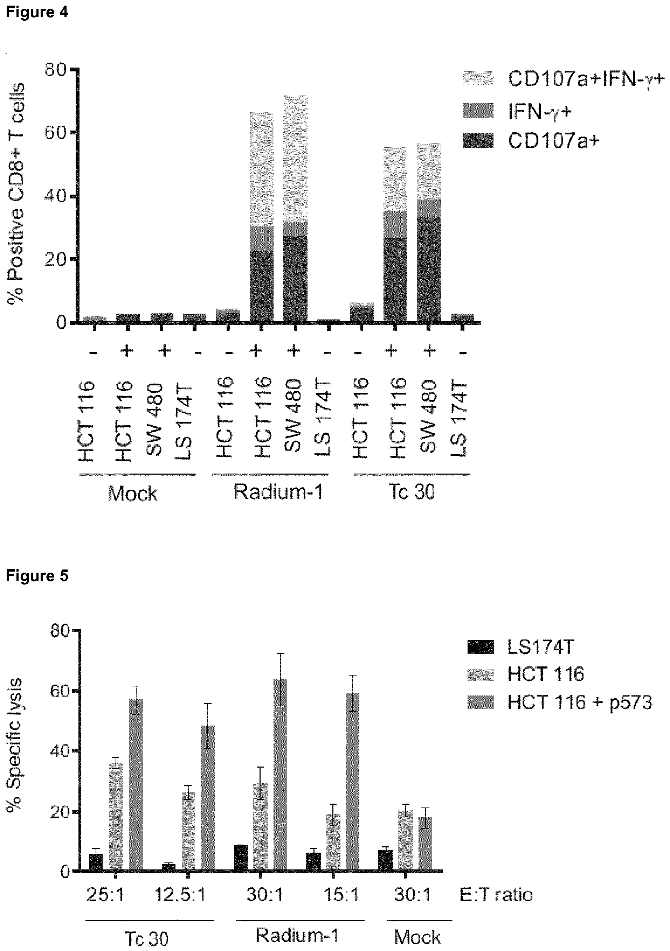

**Figure 4** shows TGFβRII^{mut}-TCR transfected CD8⁺ T-cells produce more IFN-γ and degranulate more efficiently than the original T-cell clone in response to colon cancer cell lines loaded with peptide p573. TCR-transfected CD8⁺ T-cells and the original T-cell clone were incubated with colon cancer cell lines for 6 hrs before staining of CD107a and IFN-γ was performed. The colon cancer cell lines all harbour the TGFβRII -1A frameshift mutation. HCT116 and SW480 are both HLA-A2⁺, whereas colon cancer cell line LS174T is HLA-A2⁻. Plots are CD8 gated T-cells. The results shown are representative of three independent experiments.
**Figure 5** shows TGFβRII^{mut}-TCR transfected T-cells kill target cells harbouring the TGFβRII -1A frameshift mutation with comparable efficiency to the original T-cell clone. Colon cancer cell lines LS174 and HCT116 were loaded with ⁵¹Cr. Half of the HCT116 cells were loaded with the TGFβRII frameshift peptide p573. The original patient T-cell clone, TCR-transfected CD8⁺ T-cells and mock-transfected CD8⁺ T-cells were added at E:T ratios as indicated. Cells were co-incubated for 6 hrs before ⁵¹Cr-release was measured. The results shown are representative of two independent experiments.

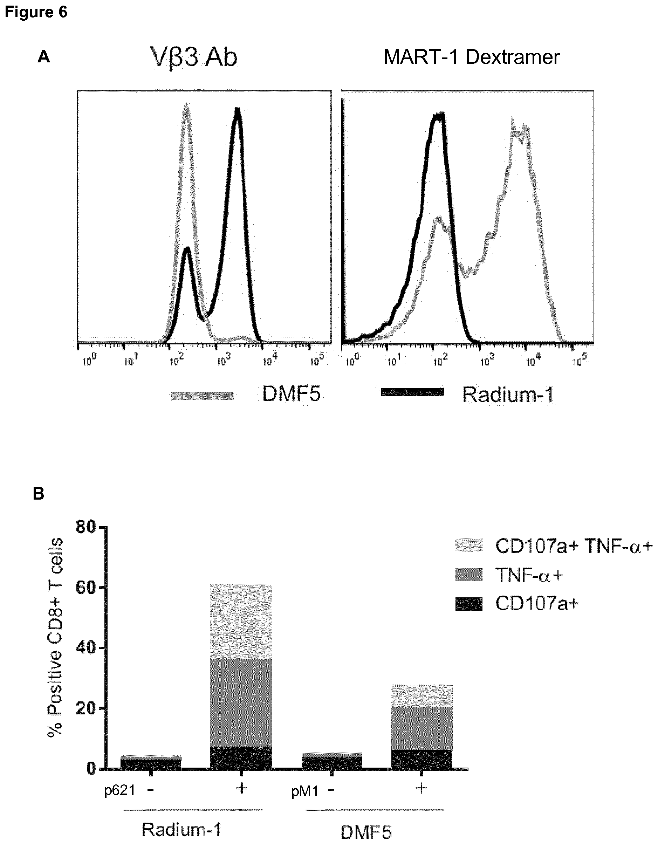

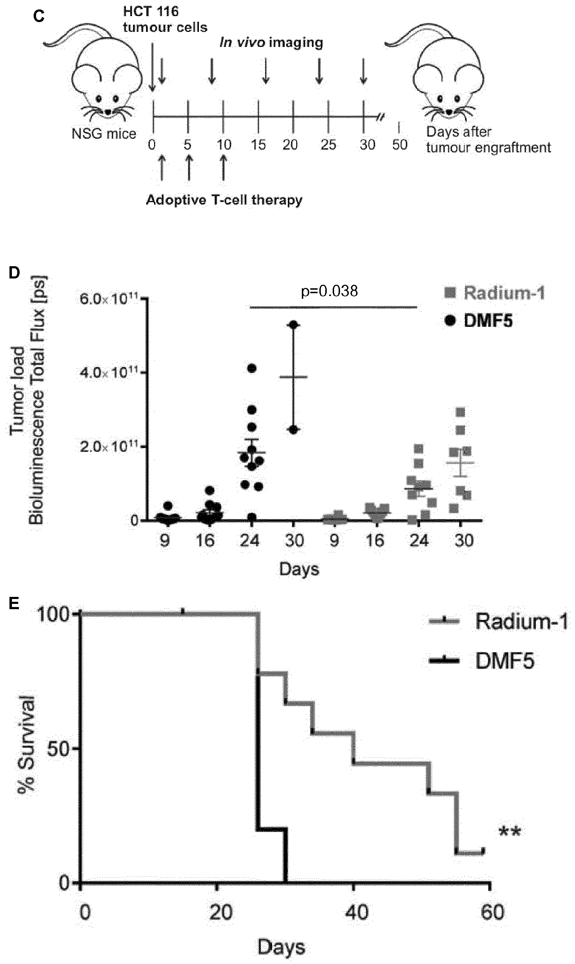

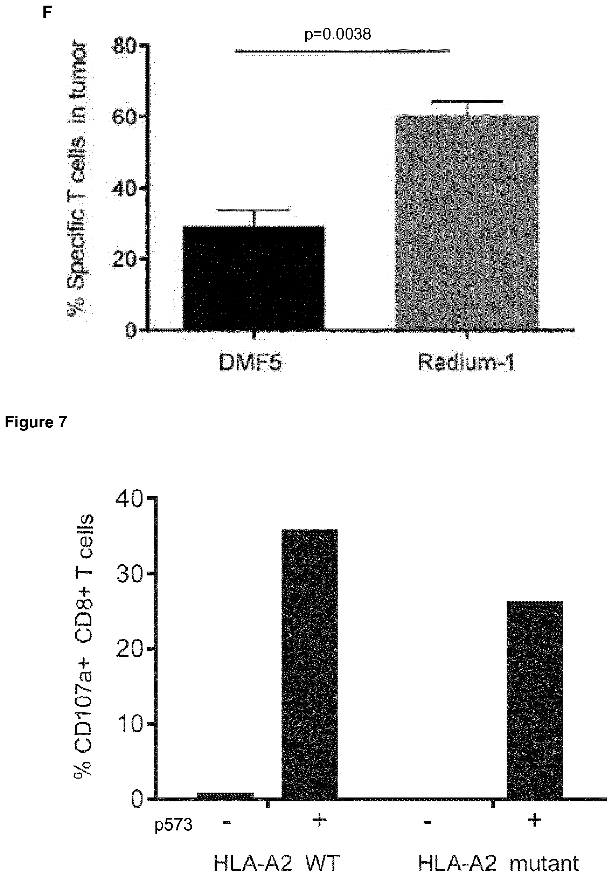

**Figure 6** shows TGFβRII^{mut}-TCR transduced T-cells are effective both *in vitro* and *in vivo.* Donor T-cells were transduced with TGFβRII^{mut}-TCR or, as a negative control, DMF5 (MART-1-specific) TCR.
   (a) shows that transduction efficiency was found to be around 60 % for each of the TCRs when T-cells were stained with either anti-Vβ3 antibody (TGFβRII^{mut}-TCR) or MART-1 dextramer (DMF5).
   In (b) the transduced T-cells were tested for reactivity against the cognate antigen before infusion. HLA-A2⁺ EBV-LCLs were loaded with either a long TGFβRII -1A frameshift mutation peptide covering the CD8⁺ T-cell epitope (p621, SEQ ID NO: 55), or the native MART-1 26-35 peptide (SEQ ID NO: 56). Transduced T-cells were co-incubated with the EBV-LCLs for 5 hours at an E:T ratio of 1:3 and stained for degranulation (CD107a) and TNF-α.
   In (c) NSG mice (TGFβRII^{mut}-TCR, n=10; MART-1 TCR, n=10) were injected intraperitoneally (i.p.) with 10⁶ HCT116 cells expressing firefly-luciferase two days before the intraperitoneal injection of 8 × 10⁶ TCR-transduced T-cells (injections on day 0 and day 2 respectively). Treatment was repeated on days 5 and 10 with 2 × 10⁷ TGFβRII^{mut}-TCR⁺ T-cells. Tumour load was evaluated by bioluminescence imaging on days 2, 9, 16, 24 and 30.
   In (d) bioluminescence signals (photons/sec) for all mice are shown in the scatter plot with mean indicated (+/- SD).
   In (e) Kaplan-Meyer analysis shows that TGFβRII^{mut}-TCR⁺ T-cell treated mice had a significantly prolonged survival compared to control mice (p=0.038; unpaired t-test). *In vivo* experiments were repeated three times and one representative experiment is shown.
   In (f) tumours were dissected from euthanized mice, single cell suspensions were made and stained for the presence of transduced T cells using anti-CD3 and either anti-Vβ3 antibody (TGFβRII^{mut}-TCR) or MART-1 dextramer (DMF5). The percentage of MART-1-specific T-cells in the control group tumours was significantly lower than the percentage of TGFβRII^{mut}-specific T-cells in tumours of mice treated with the TGFβRII^{mut}-specific TCR (p=0.0038).
**Figure 7** shows CD107a expression of TCR-transfected CD8+ T-cells is slightly reduced following stimulation with peptide-loaded HLA-A2⁺ HEK cells unable to bind CD8. HEK293 cells were transfected with mRNA encoding HLA-A2 wt or mutant HLA-A2, unable to bind CD8. HEK293 cells were loaded with peptide p573 and used to stimulate for 5 hours CD8⁺ T-cells transfected with the TGFβRII^{mut}-specific TCR.

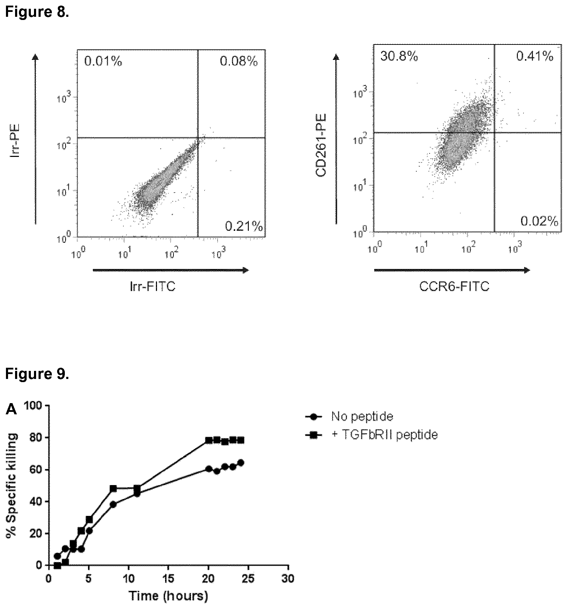

**Figure 8** shows that the colon cancer cell HCT116 expresses TRAIL-receptor 4 (CD261). The left panel shows staining of HCT116 cells with isotype controls; the right panel shows the staining of CD261 and CCR6 on HCT116 cells.

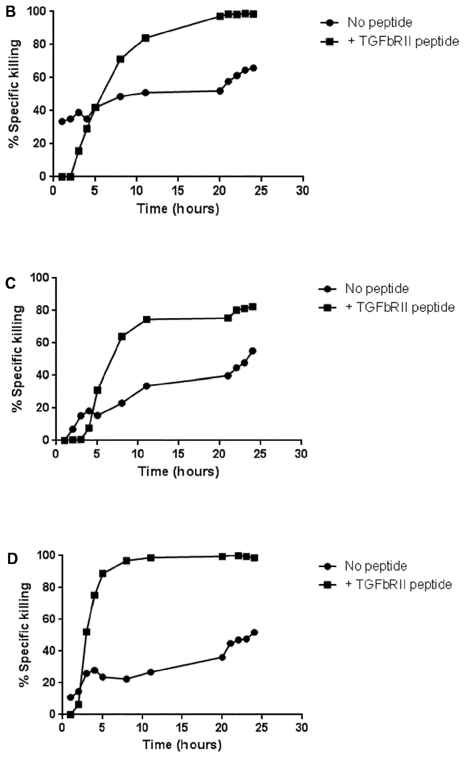

**Figure 9** shows that both CD4+ and CD8+ T-cells redirected with the Radium-1 TCR directly kill target cells presenting cognate antigen. Purified CD4+ T-cells (A) or purified CD8+ T-cells (B) electroporated with Radium-1 mRNA could kill target cells carrying both the specific TGFβRII frameshift mutation and HLA-A2 (HCT 116, colon cancer cells) ("No peptide"). Addition of exogenous long TGFβRII frameshift peptide p621 (SEQ ID NO: 55), which contains SEQ ID NO:1 (also known as sequence p573), led to increased cell killing. Purified CD4+ T-cells (C) or purified CD8+ T-cells (D) were also able to kill HLA-A2 positive cell lines (Granta; B-cell lymphoma cell line, TGFbRII frameshift negative) loaded with exogenous peptide p621. All data are representative of at least two independent experiments.

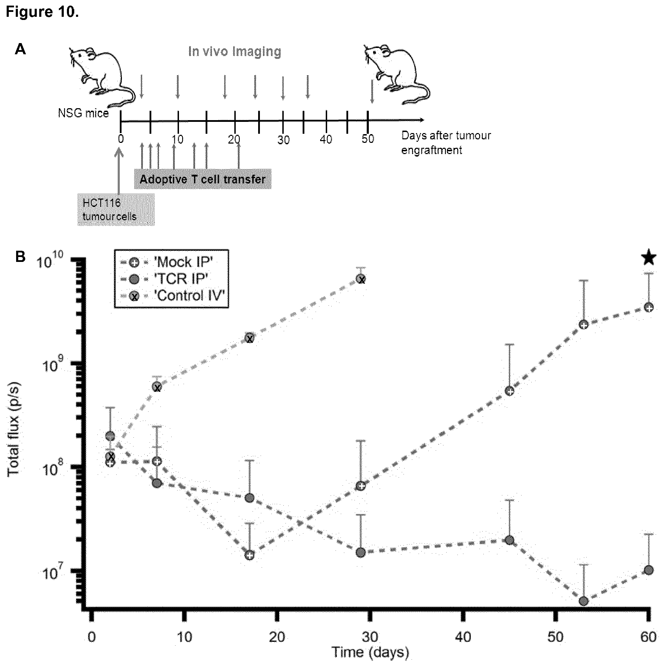

**Figure 10** shows that T-cells electroporated with Radium-1 TCR mRNA reduce *in vivo* tumour growth in mice after multiple infusions. (A) shows the experimental timeline; (B) shows tumour load for the various mouse groups (n=10 for each group) as measured by bioluminescence. Bioluminescence signals are shown as total flux (photons/sec). The means for all mice are shown in the above plot; error bars indicate standard deviation.

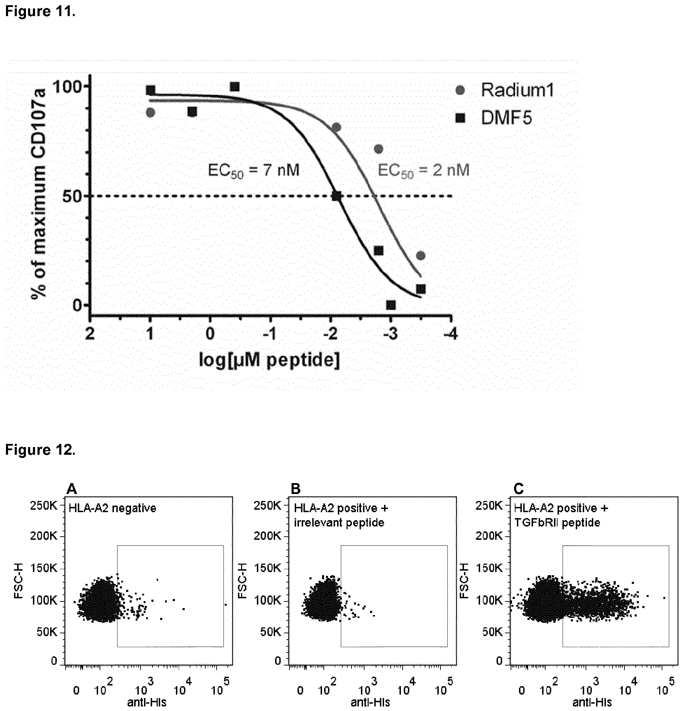

**Figure 11** compares the EC₅₀ of Radium-1 TCR-expressing CD8- T-cells relative to the EC₅₀ of DMF5 TCR-expressing CD8- T-cells, each recognising their cognate antigen. All data are representative of two independent experiments.
**Figure 12** shows results of flow cytometry of SupT1 cells to identify soluble Radium-1 TCR binding. The soluble TCR does not bind HLA-A2-negative cells (A) or HLA-A2-positive cells presenting a non-specific peptide (B). However, the soluble TCR was shown to bind HLA-A2-positive cells on which the TGFβRII frameshift peptide p573 (SEQ ID NO: 1) was presented.

### Examples

### Example 1

### Materials and Methods:

### Cell lines, Media and Reagents

A TGFβRII frameshift mutation-reactive, HLA-A2-restricted CTL (cytotoxic T-lymphocyte) clone was isolated from the blood of an MSI+ colon cancer patient and cloned by limiting dilution. The patient had been vaccinated with a 23-mer TGFβRII (-1A) frameshift peptide (a peptide with SEQ ID NO: 49). The clinical trial was approved by the Norwegian Medicines Agency, the Committee for Medical Research Ethics, Region South and the Hospital Review Board. The treatment was performed in compliance with the World Medical Association Declaration of Helsinki. Informed consent was obtained from the patient. The autologous Epstein Barr Virus-transformed lymphoblastoid cell line (EBV-LCL) was generated by transformation of B-cells from the donor. The antigen processing-deficient T2 cell line was used as a T-cell target in flow cytometry and cytotoxicity assays. Colon cancer cell lines HCT116, SW480 and LS174T as well as Human Embryonic Kidney (HEK) 293 cells were obtained from the ATCC (Rockville, MD, USA). Hek-Phoenix (Hek-P, inventors' collection) were grown in DMEM (PAA, Paschung, Austria) supplemented with 10 % HyClone FCS (HyClone, Logan, UT, USA) and 1 % antibiotic-antimicotic (penicillin/streptomycin, p/s, PAA).

Where nothing else is indicated, cells were cultured in RPMI-1640 (PAA Laboratories, Pasching, Austria) supplemented with gentamicin, 10 % heat-inactivated FCS (PAA Laboratories, Pasching, Austria). Colon cancer cell lines were treated with 500 U/ml IFN-γ (PeproTech, Rocky Hill, NJ, USA) overnight before use as target cells.

All T-cells were grown in CellGro DC medium (CellGenix, Freiburg, Germany) supplemented with 5 % heat-inactivated human serum (Trina Bioreactives AG, Nänikon, Switzerland), 10 mM N-acetylcysteine (Mucomyst, AstraZeneca AS, London, UK), 0.01 M HEPES (Life Technologies, Norway) and 0.05 mg/mL gentamycin (Garamycin, Schering-Plough Europe, Belgium), denoted complete medium hereafter, unless otherwise stated.

### Generation of T-cell lines and clones specific for TGFβRII frameshift peptides

PBMCs collected pre- and post-vaccination were available for analysis. The PBMCs had been isolated and frozen as previously described (Brunsvig, PF et al. (2006), Cancer Immunol Immunother55(12):1553-1564). Thawed PBMCs were stimulated one round *in vitro* with peptide for 10-12 days and then tested in triplicates in T-cell proliferation assays (3H-Thymidine) using autologous PBMCs as APCs. PBMCs from various time points were stimulated with TGFβRII frameshift peptides. This included peptides 573 (p573, SEQ ID NO: 1), and 621 (p621, SEQ ID NO: 55) from a TGFβRII frameshift protein resulting from a 1 bp-deletion (-1A) in an adenosine stretch (A10) from base number 709-718 of *TGFBRII.* (The GenBank sequence for wild type human *TGFBRII* is: NM 003242.) hTERT peptide I540 (SEQ ID NO: 54) was used as a negative control. Both peptides were provided by Norsk Hydro, ASA, Porsgrunn, Norway.

The MART-1 peptide with SEQ ID NO: 56 (amino acids 26-35 of native MART-1) was manufactured by Prolmmune Ltd, UK. The stimulated T-cells were then tested in proliferation assays against peptide-loaded APCs, either autologous PBMC or EBV-LCL. The Stimulation Index (SI) was defined as proliferation with peptide divided by proliferation without peptide and an SI ≥ 2 was considered a positive response. T-cell clones from responding T-cell lines were generated as previously described (Saeterdal, I et al. (2001), Cancer Immunol Immunother 50(9):469-476).

### TCR and HLA-A2 cloning

Frameshift-specific T-cell clones (26 and 30) were grown and total RNA was prepared. The cloning was performed using a modified 5'-RACE method. Briefly, cDNA was synthesized using an oligo-dT primer and was tailed at the 5'-end with a stretch of cytosines. A polyguanosine primer together with a constant domain-specific primer was used to amplify TCR chains. The amplicon was cloned and sequenced. The expression construct was prepared by amplifying TCR α- and β-chains separately with specific primers and a second PCR was performed to fuse the TCR chains as a TCR-2A construct. The TCR-2A reading frame was cloned into pENTR (Invitrogen) and subsequently recombined into other expression vectors.

For RNA synthesis the insert was sub-cloned into a Gateway modified version of pCIpA₁₀₂ (Saeboe-Larssen, S et al. (2002), J Immunol Methods 259(1-2):191-203). A detailed method as well as the primer sequences can be found in (Wälchli, S et al. (2011), PloS one 6(11):e27930). For retroviral transduction the insert was sub-cloned into the pM71 vector. The HLA-A*0201-pCIpA₁₀₂ construct was cloned as previously described (Stronen, E et al. (2009), Scand J Immunol 69(4):319-328). This construct was used as a template to generate a CD8 binding-deficient mutant by targeting the residues D227 and T228 and replacing them with K and A, respectively, as described in (Xu, XN et al. (2001), Immunity 14(5):591-602). A standard site-direct mutagenesis was performed using the following primers: 5'-GAGGACCAGACCCAGAAGGCGGAGCTCGTGGAGAC-3' (SEQ ID NO: 57) and 5'-GTCTCCACGAGCTCCGCCTTCTGGGTCTGGTCCTC-3' (SEQ ID NO: 58). HEK 293 cells were transfected with these constructs using FuGENE-6 (Roche, Switzerland) following the manufacturer's protocol.

### In vitro mRNA transcription

*In vitro* mRNA synthesis was performed essentially as previously described (Almasbak, H et al. (2011), Cytotherapy 13(5):629-640). Anti-Reverse Cap Analog (Trilink Biotechnologies Inc., San Diego, CA, USA) was used to cap the RNA. The mRNA was assessed by agarose gel electrophoresis and Nanodrop (Thermo Fisher Scientific, Waltham, MA, USA).

### In vitro expansion of human T-cells

T-cells from healthy donors were expanded using a protocol adapted for GMP production of T-cells employing Dynabeads CD3/CD28 essentially as previously described (Almasbak, H. et al. (2011), Cytotherapy 13(5):629-640). In brief, PBMCs were isolated from buffy coats by density gradient centrifugation and cultured with Dynabeads (Dynabeads^{®} *ClinExVivo*^{™} CD3/CD28, kindly provided by Dynal Invitrogen, Oslo, Norway) at a 3:1 ratio in complete CellGro DC Medium with 100 U/mL recombinant human interleukin-2 (IL-2) (Proleukin, Novartis Healthcare, USA) for 10 days. The cells were frozen and aliquots were thawed and rested in complete medium before transfection.

### Electroporation of expanded T-cells

Expanded T-cells were washed twice and resuspended in CellGro DC medium (CellGenix GmbH) and resuspended to 7 × 10⁷ cells/mL. The mRNA was mixed with the cell suspension at 100 µg/mL, and electroporated in a 4-mm gap cuvette at 500 V and with a time constant of 2 msec using a BTX 830 Square Wave Electroporator (BTX Technologies Inc., Hawthorne, NY, USA). Immediately after transfection, T-cells were transferred to complete culture medium at 37°C in 5 % CO₂ overnight to allow TCR expression.

### Antibodies and flow cytometry

T-cells were washed in staining buffer (SB) consisting of phosphate buffered saline (PBS) containing 0.1 % human serum albumin (HSA) and 0.1 % sodium azide before staining for 20 min at RT. The cells were then washed in SB and fixed in SB containing 1 % paraformaldehyde. For intracellular staining, T-cells were stimulated for 6 hours or overnight with APCs, loaded or not with p573, at a T-cell to target ratio of 2:1 and in the presence of BD GolgiPlug and BD GolgiStop at a 1/1000 dilution . Cells were stained both extracellularly and intracellularly using the PerFix-nc kit according to the manufacturer's instructions (Beckman Coulter Inc, USA). The following antibodies were used: Vβ3- FITC (Beckman Coulter-Immunotech SAS, France), CD3-eFluor 450, CD4-eFluor 450, CD4-PE-Cy7, CD8-APC, CD8-eFluor 450, CD8-PE-Cy7, CD56-PE-Cy5.5 (BD Biosciences, USA) and CD107aPE-Cy5 (BD Biosciences, USA), CXCR2-PE, IFN-γ-FITC, IL-2-APC, TNF-α-PE (BD Biosciences, USA), CD261/TRAIL-R4-PE (BD Biosciences, USA). MART-1 (aa 26-35) specific TCR was detected with dextramer staining (Immudex, Denmark) following the manufacturer's recommendations. All antibodies were purchased from eBioscience, USA, except where noted. Cells were acquired on a BD LSR II flow cytometer and the data analysed using FlowJo software (Treestar Inc., Ashland, OR, USA).

### ⁵¹Cr-release assays

⁵¹Cr-release cytotoxicity assays were performed by labelling of 2 × 10⁶ target cells in 0.5 ml FCS with Na₂⁵¹CrO₄ (7.5 MBq) (Perkin Elmer, Waltham, MA, USA), for 1 h with gentle mixing every 15 min. Cells were washed three times in cold RPMI-1640 and seeded at 2 × 10³ target cells in 96-well, U-bottomed microtitre plates. Autologous EBV-LCL, T2 target cells or colon cancer cell lines HCT116, SW480 and LS174T were pulsed with 10 µM p573 or pl540 for 1h at 37°C. The original T-cell clone, TCR-transfected T cells or mock-transfected T-cells were added at the effector-to-target (E:T) ratios indicated and the plate was left for 4 hours at 37°C as indicated. The maximum and spontaneous ⁵¹Cr release of target cells was measured after incubation with 5 % Triton X-100 (Sigma-Aldrich, Oslo, Norway) or medium, respectively. Supernatants were harvested onto Luma Plates (Packard, Meriden, CT) and ⁵¹Cr released from lysed cells was measured using a TopCount microplate scintillation counter (Packard Instrument Company, Meriden, USA). The percentage of specific chromium release was calculated by the formula: [(experimental release - spontaneous release)/(maximum release spontaneous release)] × 100.

### Retroviral transduction

PBMCs isolated from healthy donors were cultured and activated in CellGro DC medium (CellGenix GmbH, Germany) supplemented with 5 % human serum (HS) and 100 U/ml IL2 (Proleukin, Novartis Healthcare)) for 48 h in a 24-well plate precoated with anti-CD3 (OKT-3) and anti-CD28 antibodies (BD Biosciences, USA). After two days of culture PBMCs were harvested and transduced twice with retroviral supernatant. Spinoculation of PBMCs was performed with 1 volume of retroviral supernatant in a 12-well culture non-treated plate (Nunc A/S, Roskilde, Denmark) pre-coated with retronectin (20 µg/mL, Takara Bio. Inc., Shiga, Japan). After two days, cells were harvested with PBS-EDTA (0.5 mM). Transduced T-cells were further expanded using Dynabeads CD3/CD28 as described above.

### Mouse xenograft studies

NOD.Cg-Prkdc^{scid} II2rg^{tm1Wjl}/SzJ (NSG) mice were bred in-house under an approved institutional animal care protocol and maintained under pathogen-free conditions. 6-8 week-old mice were injected i.p. with 1-1.5 × 10⁶ HCT116 tumour cells. The HCT116 cells were engineered with a retroviral vector (provided by Dr. Rainer Löw, EUFETS AG, Idar-Oberstein, Germany) to express firefly luciferase and EGFP. Tumour growth was monitored by bioluminescent imaging using the Xenogen Spectrum system and Living Image v3.2 software. Anaesthetised mice were injected i.p. with 150 mg/kg body-weight of D-luciferin (Caliper Life Sciences, Hopkinton, MA). Animals were imaged 10 minutes after luciferin injection.

### Statistical analysis

Continuous data were described with median, mean and range. The Mann Whitney test was used for analysis of tumour load, while survival was calculated using the Kaplan Meier method with the unpaired t-test used for comparison of survival between groups. All p-values given are two-tailed values. A p-value below 0.05 was considered significant. All statistical analyses were performed using *GraphPad Prism*^{®} (GraphPad Software, Inc. USA).

### Results

### Isolation of a TGFβRII frameshift mutation-specific T-cell clone

A TGFβRII frameshift mutation-reactive, HLA-A2-restricted CTL was isolated from the blood of an MSI+ colon cancer patient. The patient had been vaccinated with a 23-mer TGFβRII frameshift peptide of SEQ ID NO: 49. The CTL clones were shown to be CD8⁻CD4⁻ and about 50 % of the cells expressed CD56 (Fig. 1a). The CTL clones were previously suspected to be monoclonal since they were shown to express TCR Vβ3 (or TRBV 28, IMGT nomenclature) (Kyte, JA (2009), Expert Opin Investig Drugs 18(5):687-694). The molecular cloning revealed that they were indeed sister clones, harbouring the same pair of TCR chains. Specific lysis of the colon cancer cell lines HCT116 and SW480 in the absence of exogenously loaded peptide was observed. However, the E:T ratio required for lysis of cell lines with endogenous peptide was higher than if cell lines were loaded exogenously with TGFβRII frameshift peptide (p573, SEQ ID NO: 1). As a control, another colon cancer cell line, LS174T, which is HLA-A2 negative but expresses the TGFβRII mutation was not killed (Fig. 1b). Importantly, despite the expression of CD56 on the T-cell clone (Fig. 1a), the HLA-A2 negative LS174T cell line was not killed, indicating that the killing was not mediated by natural killer (NK)-cell-like activity, but by specific recognition of MHC molecules loaded with peptide.

To test the relative avidity of the T-cell clones, TAP-deficient T2 cells were loaded with titrated amounts of peptide (0.01-1.0 µM). We observed that the killing activity followed the peptide concentration (Fig. 1c). The addition of HLA-specific blocking antibodies reduced the killing (Fig. 1c), supporting the HLA class I restriction of the TCR. Similar observations were made when autologous EBV-LCLs were used as APCs. Taken together, the data show that the TGFβRII^{mut}-specific T-cells were co-receptor negative, peptide-specific and HLA class I-restricted.

### TGFβRII^{mut}-TCR is expressed and active in both CD4⁺ and T-cells following mRNA electroporation

The TCR α- and β-chains from the TGFβRII^{mut}-reactive T-cell sister clones were identified and referred to hereafter as the Radium-1 TCR. We cloned the two chains into an mRNA expression vector (see Materials and Methods) and 10-day *in vitro*-expanded T-cells were electroporated in order to assess their ability to recognize their targets. Radium-1 TCR expression was measured in both CD4⁺ and CD8⁺ T-cells by surface staining using an anti-Vβ3 (TRBV 28) antibody (Fig. 2a). Around 70 % of transfected T cells expressed the Vβ3 chain, with 42 % of these T-cells being CD8 positive and 32 % of T-cells expressing CD4, whereas less than 5 % of the cells naturally express Vβ3.

We then monitored the activity of Radium-1-transfected T-cells by intracellular cytokine staining upon co-incubated with the colon cancer cell lines SW480 and LS174T. Colon cancer cell line SW480 was recognised by both CD8⁺ and CD4⁺ T-cells in the absence and presence of exogenously loaded peptide. The T-cells produced TNF-α and IFN-γ (Fig. 3). As expected, the colon cancer cell line LS174T was not recognized. These data confirmed the HLA-peptide restriction of the Radium-1 TCR and its ability to efficiently redirect both CD4⁺ and CD8⁺ T-cells.

### CD107a and IFN-γ production in Radium-1 TCR-transfected CD8⁺ T-cells

To determine the cytotoxic potential of TCR-transfected CD8⁺ T-cells against colon cancer cell lines, mRNA-electroporated T-cells were co-incubated with the colon cancer cell lines for 6 hrs and stained with antibodies against the degranulation marker CD107a and IFN-γ (Fig. 4). Very low levels of IFN-γ production and CD107a expression were detected in the absence of exogenously loaded peptide. Upon the addition of peptide p573 (SEQ ID NO: 1), both Radium-1 TCR-transfected T-cells and the original T-cell clone were strongly activated. Interestingly, TCR-transfected T-cells were more efficient IFN-γ producers and also displayed higher levels of degranulation than the original T-cell clone, whereas mock-transfected T-cells were not activated. To test the co-receptor independency of the TCR HEK293 cells were transfected with either wild type (wt) HLA-A2 or mutant HLA-A2 unable to bind CD8, loaded with p573 and used to stimulate TCR-transfected CD8⁺ T-cells. The number of T-cells expressing CD107a was 36 % (wt HLA-A2) and 26 % (mutant HLA-A2), indicating that this TCR is at least partially co-receptor independent (Fig. 7).

### Radium-1 TCR-transfected T-cells are capable of mediating specific tumour cell lysis

In addition to cytokine production, the main function required of adoptively transferred redirected T-cells is to specifically kill tumour cells. To investigate if the TCR-transfected T-cells were capable of target-cell lysis, they were tested against the colon cancer cell lines in 6-hr chromium-release assays (Fig. 5). TCR-transfected T-cells lysed HCT116 cells at levels comparable to the original patient clone. As expected, the lysis was further increased when exogenous p573 (SEQ ID NO: 1) was added. The lysis of HLA-A2 negative cell line LS174T was similar to that of mock-transfected T-cells, demonstrating low background lysis of HCT116 likely due to TRAIL-R expression on the target cells (Fig. 8). This cell line has been reported by others to be sensitive to TRAIL-mediated lysis (Tang, W et al. (2009), Febs J 276(2):581-593).

### Radium-1-TCR-transduced T-cells are effective in vitro and in vivo

We established a xenograft mouse model of colon cancer by intraperitoneal injection of HCT116 cells (Kishimoto, H et al. (2009), Proc Natl Acad Sci U S A 106(34):14514-14517). T-cells were retrovirally transduced with TCR and tested for expression, which was around 60 % for both the Radium-1 TCR and the MART-1-specific TCR (DMF5) used as a control (Fig. 6a). Prior to injection, T-cells were tested functionally against HLA-A2⁺ EBV-LCLs loaded with either a long TGFβRII frameshift peptide (p621, SEQ ID NO: 55) or low affinity (wt) MART-1 peptide (SEQ ID NO: 56) (Fig. 6b). The T-cells expressing the Radium-1 TCR all responded against EBV-LCLs loaded with the long TGFβRII frameshift peptide, while around half of the MART-1 TCR expressing cells responded against the low affinity MART-1 peptide. NSG mice were injected i.p. with 10⁶ HCT116 cells on day 0 and on d2, d5 and d10 mice were injected with 8 × 10⁶ (d2) and 2 × 10⁷ (d5 & d10) T-cells (Fig. 6c). Control mice were treated with T-cells expressing the MART-1 specific TCR.

*In vivo* live imaging of the mice showed that the tumour load was significantly lower (p=0.038) in mice that received the treatment with TGFβRII^{mut}-specific T-cells compared to the MART-1-specific control T-cells (Fig. 6d). The mice receiving TGFβRII^{mut}-specific T-cells also had enhanced survival compared to control mice (p=0.038, Fig. 6e). Tumours were dissected from mice that had to be euthanised due to high tumour load. Single cell suspensions of the tumours were made and stained with anti-human CD3 and anti-Vβ3 or MART-1 dextramer. The percentage of TCR-expressing T-cells in the tumour was found to be significantly higher in mice who received the treatment with TGFβRII^{mut}-specific T-cells (p=0.0038, Fig. 6f) despite the transduction efficiency of the two T-cell populations being very similar, indicating that the TGFβRII^{mut}-specific T-cells are either recruited to the tumour more efficiently or that they proliferate *in vivo* due to antigenic stimulation. Taken together, these data demonstrate the pre-clinical potency of Radium-1 TCR *in vivo.*

### Example 2

To investigate target cell killing by CD4+ and CD8+ T-cells transduced with the Radium-1 TCR, target cells were stably-transduced to express luciferase. Two sets of target cells were used: the HCT116 cell line and the Granta cell line. HCT116 cells are described above; the Granta cell line is a human B-cell lymphoma cell line. Changes in bioluminescence were used to measure changes in target cell number during culture with the Radium-1-transduced T-cells, representing killing of the target cells by the T-cells.

Luciferase-transduced target cells were co-cultured with effector T-cells at an effector to target (E:T) ratio of 30:1, and bioluminscence measured. The cells were co-cultured for 24 hours, and bioluminescence measured at 1, 2, 3, 4, 5, 8, 11, 20, 21, 22, 23 and 24 hrs. Effector T-cells were co-cultured with Granta cells both with and without exogenous peptide p621 (SEQ ID NO: 55), which comprises the sequence of SEQ ID NO: 1.

Purified CD4+ T-cells and purified CD8+ T-cells transduced with Radium-1 mRNA were both found to kill both HCT116 cells and Granta cells (Fig. 9). Killing of Granta cells by both CD4+ and CD8+ T-cells was significantly higher in the presence of p621 ("+ TGFβRII peptide") than in its absence ("no peptide"). This demonstrates that Radium-1-transduced CD4+ T-cells are able to kill target cells without interaction with CD8+ T-cells.

The *in vivo* killing activity of T-cells transiently transduced with Radium-1 was further investigated in mice. NSG mice were injected i.p. with 10⁶ HCT116 cells stably transduced to express luciferase. Two days later (i.e. on day 2) the mice were injected i.p. or i.v. (intravenously) with 8-10 × 10⁶ Radium-1-transfected T-cells. Further injections of Radium-1-transfected T-cells were administered on days 5, 7, 10, 13, 15 and 21, and tumour load was evaluated by bioluminescence imaging on days 2, 7, 17, 29, 45, 53 and 60 (see Fig. 10A; final imaging not indicated).

Mice treated with Radium-1 TCR transfected T-cells showed a significantly lower tumour load than those treated with mock-transfected T-cells (Fig. 10B) (*p=0.01, Wilcoxon Mann Whitney test). Due to T-cell alloreactivity, mock-transfected T-cells had some effect on tumour growth after multiple injections, as shown. However, this effect was only temporary. As TCR expression was transient in this case, T-cells injected intravenously (i.v.) showed no effect on tumour growth.

The effectiveness of the Radium-1 TCR was compared *in vitro* to a known high-affinity TCR. The MART-1-specific TCR DMF5 was selected for comparison. DMF5 has been used clinically in the treatment of melanoma (Johnson, L.A. et al. (2006), J Immunol 177(9):6548-6559).

CD8- T-cells were transduced with Radium-1 and MART-1 and sorted. TCR+ T-cells were incubated with HLA-A2+ T2 cells (T2 is a human lymphoblast cell line which does not express Class II MHC molecules) loaded with the TGFβRII frameshift peptide p573 (SEQ ID NO: 1) and the MART-1 26-35 peptide analogue ELAGIGILTV (SEQ ID NO: 80) for 5 hours before staining for the degranulation marker CD107a as a marker of killing capacity followed by flow cytometry analysis. The MART-1 26-35 peptide analogue of SEQ ID NO: 80 has a single amino acid substitution relative to the wild-type peptide with SEQ ID NO: 56, i.e. the alanine at position 2 of SEQ ID NO: 56 is substituted for a leucine. The resultant analogue peptide has advantageous properties, in that it has enhanced affinity for HLA-A2, leading to enhanced presentation of the peptide by HLA-A2-containing Class I MHC molecules compared to the wild-type peptide.

The EC₅₀ of p573 for Radium-1 was shown to be 2 nM, compared with a value of 7 nm for the MART-1 peptide of SEQ ID NO: 56 with DMF5 (Fig. 11). This indicates that Radium-1 has very high affinity for its cognate antigen/MHC complex, and CD8-independent. Furthermore, Radium-1 is shown to have a higher affinity for its cognate antigen/MHC complex than the DMF5 TCR, which is known to be clinically effective.

### Example 3

The soluble, His-tagged Radium-1 TCR encoded by the scTCR of SEQ ID NO: 69 was expressed in HEK cells. The supernatant of the expressing HEK cells was isolated. SupT1 cells (an HLA-A2-negative cell line) were transduced to express HLA-A2, either fused to a non-specific, irrelevant peptide not recognised by Radium-1, or to a TGFβRII frameshift peptide. The transduced cells were incubated for 30 mins at room temperature with the soluble Radium-1 TCR; untransduced cells were also incubated with the soluble TCR as a further negative control. After incubation, the cells were washed and then stained with allophycocyanin (APC) to identify soluble TCR binding. Staining was performed using a primary mouse anti-His antibody followed by a secondary APC-conjugated anti-mouse IgG antibody. Stained cells were then analysed by flow cytometry (Fig. 12).

As shown in Fig. 12A and 12B, essentially no staining of the negative controls was seen, demonstrating that the soluble Radium-1 TCR does not bind cells which do not express HLA-A2, or which express HLA-A2 but are not presenting the TGFβRII frameshift peptide. Fig. 12C shows that cells expressing HLA-A2 and presenting the TGFβRII frameshift peptide were recognised by the soluble Radium-1 TCR, showing it has the expected specificity.

## Claims

1. A nucleic acid molecule encoding a T-cell receptor (TCR) molecule directed against a mutated TGFβRII protein which comprises the sequence of SEQ ID NO: 1, wherein said TCR molecule is capable of binding a peptide of SEQ ID NO: 1 when said peptide is presented by a Class I Major Histocompatibility Complex (MHC) comprising HLA-A2, wherein said TCR molecule, when expressed by an immune effector cell, is located on the surface of the cell, and wherein said TCR molecule comprises an α-chain domain and a β-chain domain,
wherein said α-chain domain comprises:
i) a variable region comprising the amino acid sequence set forth in SEQ ID NO: 8; and
ii) a constant region comprising the amino acid sequence set forth in SEQ ID NO: 9, or a murinised version of SEQ ID NO: 9; and
said β-chain domain comprises:
i) a variable region comprising the amino acid sequence set forth in SEQ ID NO: 13; and
ii) a constant region comprising the amino acid sequence set forth in SEQ ID NO: 14, or a murinised version of SEQ ID NO: 14.

2. The nucleic acid molecule of claim 1, wherein the α-chain domain comprises the amino acid sequence of SEQ ID NO: 11 and the β-chain domain comprises the amino acid sequence of SEQ ID NO: 16.

3. The nucleic acid molecule of claim 1, wherein the α-chain domain comprises the amino acid sequence of SEQ ID NO: 25 and the β-chain domain comprises the amino acid sequence of SEQ ID NO: 31

4. A nucleic acid molecule encoding a soluble T-cell receptor (TCR) molecule directed against a mutated TGFβRII protein which comprises the sequence of SEQ ID NO: 1, wherein said TCR molecule is capable of binding a peptide of SEQ ID NO: 1 when said peptide is presented by a Class I Major Histocompatibility Complex (MHC) comprising HLA-A2, and wherein said soluble TCR molecule comprises an α-chain domain and a β-chain domain,
wherein said α-chain domain comprises:
i) a variable region comprising the amino acid sequence set forth in SEQ ID NO: 72; and
ii) a constant region comprising the amino acid sequence set forth in SEQ ID NO: 60 or SEQ ID NO: 61; and
said β-chain domain comprises:
i) a variable region comprising the amino acid sequence set forth in SEQ ID NO: 75; and
ii) a constant region comprising the amino acid sequence set forth in SEQ ID NO: 62 or SEQ ID NO: 63.

5. The nucleic acid molecule of claim 4, wherein:
(i) said α-chain domain comprises the amino acid sequence set forth in SEQ ID NO: 73 and said β-chain domain comprises the amino acid sequence set forth in SEQ ID NO: 76; or
(ii) said α-chain domain comprises the amino acid sequence set forth in SEQ ID NO: 74, and said β-chain domain comprises the amino acid sequence set forth in SEQ ID NO: 77.

6. The nucleic acid molecule of any one of claims 1 to 5, wherein the TCR molecule is encoded as a single-chain TCR (scTCR) comprising an α-chain domain joined to a β-chain domain by a self-splicing linker, preferably wherein said linker is a 2A peptide and comprises the amino acid sequence of SEQ ID NO: 18, or an amino acid sequence having at least 40 % sequence identity thereto.

7. A vector comprising the nucleic acid molecule of any one of claims 1 to 6.

8. A TCR molecule, wherein said TCR molecule is:
i) a full-length TCR as defined in any one of claims 1 to 3; or
ii) a soluble TCR molecule as defined in claim 4 or 5.

9. A host cell comprising the nucleic acid molecule of any one of claims 1 to 6 or the vector of claim 7.

10. The host cell of claim 9, wherein said host cell is an immune effector cell which expresses a TCR as defined in any one of claims 1 to 3.

11. A composition comprising the soluble TCR molecule of claim 8 or the immune effector cell of claim 10;
and at least one physiologically acceptable carrier or excipient.

12. A soluble TCR molecule as defined in claim 8; an immune effector cell as defined in claim 10, or a composition as defined in claim 11, for use in therapy.

13. A soluble TCR molecule as defined in claim 8; an immune effector cell as defined in claim 10, or a composition as defined in claim 11, for use in the treatment of cancer, wherein said cancer expresses a mutated TGFβRII protein which comprises SEQ ID NO: 1.

14. The TCR molecule, immune effector cell or composition for use according to claim 13, wherein the cancer is colorectal cancer and/or said cancer is in a subject with Lynch Syndrome.

15. An *in vitro* or *ex vivo* method of generating a TGFβRII frameshift mutant-specific immune effector cell, said method comprising introducing a nucleic acid molecule as defined in any one of claims 1 to 3 or a vector as defined in claim 7, said vector comprising a nucleic acid molecule as defined in any one of claims 1 to 3, into an immune effector cell;
optionally wherein said method further comprises stimulating the cells and inducing them to proliferate before and/or after introducing the nucleic acid molecule or vector.

## Patentansprüche

1. Nukleinsäuremolekül, das für ein T-Zell-Rezeptor- (TCR) Molekül codiert, das gegen ein mutiertes TGFβRII-Protein gerichtet ist, das die Sequenz von SEQ ID NO: 1 umfasst, wobei das TCR-Molekül fähig ist, ein Peptid von SEQ ID NO: 1 zu binden, wenn das Peptid von einem Klasse-I-Haupthistokompatibilitätskomplex (MHC) präsentiert wird, der HLA-A2 umfasst, wobei das TCR-Molekül, wenn es von einer Immuneffektorzelle exprimiert wird, sich an der Oberfläche der Zelle befindet, und wobei das TCR-Molekül eine α-Kettendomäne und eine β-Kettendomäne umfasst,
wobei die α-Kettendomäne umfasst:
i) eine variable Region, die die in SEQ ID NO: 8 dargelegte Aminosäuresequenz umfasst; und
ii) eine konstante Region, die die in SEQ ID NO: 9 dargelegte Aminosäuresequenz oder eine murinisierte Version von SEQ ID NO: 9 umfasst; und
die β-Kettendomäne umfasst:
i) eine variable Region, die die in SEQ ID NO: 13 dargelegte Aminosäuresequenz umfasst; und
ii) eine konstante Region, die die in SEQ ID NO: 14 dargelegte Aminosäuresequenz oder eine murinisierte Version von SEQ ID NO: 14 umfasst.

2. Nukleinsäuremolekül nach Anspruch 1, wobei die α-Kettendomäne die Aminosäuresequenz von SEQ ID NO: 11 umfasst und die β-Kettendomäne die Aminosäuresequenz von SEQ ID NO: 16 umfasst.

3. Nukleinsäuremolekül nach Anspruch 1, wobei die α-Kettendomäne die Aminosäuresequenz von SEQ ID NO: 25 umfasst und die β-Kettendomäne die Aminosäuresequenz von SEQ ID NO: 31 umfasst.

4. Nukleinsäuremolekül, das für ein lösliches T-Zell-Rezeptor- (TCR) Molekül codiert, das gegen ein mutiertes TGFβRII-Protein gerichtet ist, das die Sequenz von SEQ ID NO: 1 umfasst, wobei das TCR-Molekül fähig ist, ein Peptid von SEQ ID NO: 1 zu binden, wenn das Peptid von einem Klasse-I-Haupthistokompatibilitätskomplex (MHC) präsentiert wird, der HLA-A2 umfasst, und wobei das lösliche TCR-Molekül eine α-Kettendomäne und eine β-Kettendomäne umfasst,
wobei die α-Kettendomäne umfasst:
i) eine variable Region, die die in SEQ ID NO: 72 dargelegte Aminosäuresequenz umfasst; und
ii) eine konstante Region, die die in SEQ ID NO: 60 oder SEQ ID NO: 61 dargelegte Aminosäuresequenz umfasst; und
die β-Kettendomäne umfasst:
i) eine variable Region, die die in SEQ ID NO: 75 dargelegte Aminosäuresequenz umfasst; und
ii) eine konstante Region, die die in SEQ ID NO: 62 oder SEQ ID NO: 63 dargelegte Aminosäuresequenz umfasst.

5. Nukleinsäuremolekül nach Anspruch 4, wobei:
(i) die α-Kettendomäne die in SEQ ID NO: 73 dargelegte Aminosäuresequenz umfasst und die β-Kettendomäne die in SEQ ID NO: 76 dargelegte Aminosäuresequenz umfasst; oder
(ii) die α-Kettendomäne die in SEQ ID NO: 74 dargelegte Aminosäuresequenz umfasst und die β-Kettendomäne die in SEQ ID NO: 77 dargelegte Aminosäuresequenz umfasst.

6. Nukleinsäuremolekül nach einem der Ansprüche 1 bis 5, wobei das TCR-Molekül als ein Einzelketten-TCR (scTCR) codiert ist, umfassend eine α-Kettendomäne, die über einen Linker mit autokatalytischer Spleißung an einer β-Kettendomäne gefügt ist, wobei der Linker vorzugsweise ein 2A-Peptid ist und die Aminosäuresequenz von SEQ ID NO: 18 oder eine Aminosäuresequenz mit mindestens 40 % Sequenzidentität zu dieser umfasst.

7. Vektor, umfassend das Nukleinsäuremolekül nach einem der Ansprüche 1 bis 6.

8. TCR-Molekül, wobei das TCR-Molekül Folgendes ist:
i) ein TCR mit voller Länge wie in einem der Ansprüche 1 bis 3 definiert; oder
ii) ein lösliches TCR-Molekül wie in Anspruch 4 oder 5 definiert.

9. Wirtszelle, umfassend das Nukleinsäuremolekül nach einem der Ansprüche 1 bis 6 oder den Vektor nach Anspruch 7.

10. Wirtszelle nach Anspruch 9, wobei die Wirtszelle eine Immuneffektorzelle ist, die ein TCR wie in einem der Ansprüche 1 bis 3 definiert exprimiert.

11. Zusammensetzung, umfassend das lösliche TCR-Molekül nach Anspruch 8 oder die Immuneffektorzelle nach Anspruch 10;
und mindestens einen physiologisch verträglichen Träger oder Hilfsstoff.

12. Lösliches TCR-Molekül wie in Anspruch 8 definiert; Immuneffektorzelle wie in Anspruch 10 definiert, oder Zusammensetzung wie in Anspruch 11 definiert, zur Verwendung bei einer Therapie.

13. Lösliches TCR-Molekül, wie in Anspruch 8 definiert; Immuneffektorzelle wie in Anspruch 10 definiert, oder Zusammensetzung wie in Anspruch 11 definiert, zur Verwendung bei der Behandlung einer Krebserkrankung, wobei die Krebserkrankung ein mutiertes TGFβRII-Protein exprimiert, das SEQ ID NO: 1 umfasst.

14. TCR-Molekül, Immuneffektorzelle oder Zusammensetzung zur Verwendung nach Anspruch 13, wobei die Krebserkrankung eine kolorektale Krebserkrankung ist und/oder die Krebserkrankung in einem Subjekt mit Lynch-Syndrom vorliegt.

15. *In-vitro-* oder *Ex*-*vivo*-Verfahren zum Erzeugen einer für TGFβRII-Rasterschubmutanten spezifischen Immuneffektorzelle, wobei das Verfahren umfasst, ein wie in einem der Ansprüche 1 bis 3 definiertes Nukleinsäuremolekül oder einen wie in Anspruch 7 definierten Vektor in eine Immuneffektorzelle einzusetzen, wobei der Vektor ein wie in einem der Ansprüche 1 bis 3 definiertes Nukleinsäuremolekül umfasst;
wobei das Verfahren wahlweise umfasst, die Zellen zu stimulieren und diese zu veranlassen, sich vor und/oder nach Einsetzen des Nukleinsäuremoleküls oder Vektors zu vermehren.

## Revendications

1. Molécule d'acide nucléique codant pour une molécule du récepteur des lymphocytes T (TCR) dirigée contre une protéine TGFβRII mutée qui comprend la séquence SEQ ID NO : 1, dans laquelle ladite molécule TCR est apte à se lier à un peptide de SEQ ID NO : 1 lorsque ledit peptide est présenté par un complexe majeur d'histocompatibilité (CMH) de classe I comprenant HLA-A2, dans laquelle ladite molécule TCR, lorsqu'elle est exprimée par une cellule effectrice immunitaire, est située à la surface de la cellule, et dans laquelle ladite molécule TCR comprend un domaine de chaîne α et un domaine de chaîne β,
dans laquelle ledit domaine de chaîne α comprend :
i) une région variable comprenant la séquence d'acides aminés définie dans SEQ ID NO : 8 ; et
ii) une région constante comprenant la séquence d'acides aminés définie dans SEQ ID NO : 9, ou une version murinisée de SEQ ID NO : 9 ; et
ledit domaine de chaîne β comprend :
i) une région variable comprenant la séquence d'acides aminés définie dans SEQ ID NO : 13 ; et
ii) une région constante comprenant la séquence d'acides aminés définie dans SEQ ID NO : 14, ou une version murinisée de SEQ ID NO : 14.

2. Molécule d'acide nucléique selon la revendication 1, dans laquelle le domaine de chaîne α comprend la séquence d'acides aminés de SEQ ID NO : 11 et le domaine de chaîne β comprend la séquence d'acides aminés de SEQ ID NO : 16.

3. Molécule d'acide nucléique selon la revendication 1, dans laquelle le domaine de chaîne α comprend la séquence d'acides aminés de SEQ ID NO : 25 et le domaine de chaîne β comprend la séquence d'acides aminés de SEQ ID NO : 31.

4. Molécule d'acide nucléique codant pour une molécule du récepteur des lymphocytes T (TCR) soluble dirigée contre une protéine TGFβRII mutée qui comprend la séquence de SEQ ID NO : 1, dans laquelle ladite molécule TCR est apte à se lier à un peptide de SEQ ID NO : 1 lorsque ledit peptide est présenté par un complexe majeur d'histocompatibilité (CMH) de classe I comprenant HLA-A2, et dans laquelle ladite molécule TCR soluble comprend un domaine de chaîne α et un domaine de chaîne β,
dans laquelle ledit domaine de chaîne α comprend :
i) une région variable comprenant la séquence d'acides aminés définie dans SEQ ID NO : 72 ; et
ii) une région constante comprenant la séquence d'acides aminés définie dans SEQ ID NO : 60 ou SEQ ID NO : 61 ; et
ledit domaine de chaîne β comprend :
i) une région variable comprenant la séquence d'acides aminés définie dans SEQ ID NO : 75 ; et
ii) une région constante comprenant la séquence d'acides aminés définie dans SEQ ID NO : 62 ou SEQ ID NO : 63.

5. Molécule d'acide nucléique selon la revendication 4, dans laquelle :
(i) ledit domaine de chaîne α comprend la séquence d'acides aminés décrite dans SEQ ID NO : 73 et ledit domaine de chaîne β comprend la séquence d'acides aminés définie dans SEQ ID NO : 76 ; ou
(ii) ledit domaine de chaîne α comprend la séquence d'acides aminés décrite dans SEQ ID NO : 74, et ledit domaine de chaîne β comprend la séquence d'acides aminés définie dans SEQ ID NO : 77.

6. Molécule d'acide nucléique selon l'une quelconque des revendications 1 à 5, dans laquelle la molécule TCR est codée en tant que TCR à chaîne unique (scTCR) comprenant un domaine de chaîne α lié à un domaine de chaîne β par un lieur d'auto-épissage, de préférence dans laquelle ledit lieur est un peptide 2A et comprend la séquence d'acides aminés de SEQ ID NO : 18, ou une séquence d'acides aminés présentant au moins 40 % d'identité de séquence avec celle-ci.

7. Vecteur comprenant la molécule d'acide nucléique selon l'une quelconque des revendications 1 à 6.

8. Molécule TCR, dans laquelle ladite molécule TCR est :
i) un TCR pleine longueur tel que défini dans l'une quelconque des revendications 1 à 3 ; ou
ii) une molécule TCR soluble telle que définie en revendication 4 ou 5.

9. Cellule hôte comprenant la molécule d'acide nucléique selon l'une quelconque des revendications 1 à 6 ou le vecteur selon la revendication 7.

10. Cellule hôte selon la revendication 9, dans laquelle ladite cellule hôte est une cellule effectrice immunitaire qui exprime un TCR tel que défini dans l'une quelconque des revendications 1 à 3.

11. Composition comprenant la molécule TCR soluble selon la revendication 8 ou la cellule effectrice immunitaire selon la revendication 10 ;
et au moins un support ou un excipient physiologiquement acceptable.

12. Molécule TCR soluble telle que définie en revendication 8; cellule effectrice immunitaire telle que définie en revendication 10, ou composition telle que définie en revendication 11, pour utilisation en thérapie.

13. Molécule TCR soluble telle que définie en revendication 8; cellule effectrice immunitaire telle que définie en revendication 10, ou composition telle que définie en revendication 11, pour utilisation dans le traitement du cancer, dans laquelle ledit cancer exprime une protéine TGFβRII mutée qui comprend SEQ ID NO : 1.

14. Molécule TCR, cellule effectrice immunitaire ou composition pour utilisation selon la revendication 13, dans laquelle le cancer est un cancer colorectal et/ou ledit cancer est présent chez un sujet atteint du syndrome de Lynch.

15. Procédé *in vitro* ou *ex vivo* de génération d'une cellule effectrice immunitaire spécifique de la forme mutante de déphasage de TGFβRII, ledit procédé comprenant l'introduction d'une molécule d'acide nucléique telle que définie dans l'une quelconque des revendications 1 à 3 ou d'un vecteur tel que défini en revendication 7, ledit vecteur comprenant une molécule d'acide nucléique telle que définie dans l'une quelconque des revendications 1 à 3, dans une cellule effectrice immunitaire ;
éventuellement dans lequel ledit procédé comprend en outre la stimulation des cellules et l'induction de leur prolifération avant et/ou après l'introduction de la molécule d'acide nucléique ou du vecteur.
